# EUROPEAN PATENT APPLICATION

(11) **EP 2 009 093 A2**
(43) Date of publication of application: **31.12.2008**
(21) Application number: 08166012.8
(22) Date of filing: 21.09.2005
(51) Int. Cl.: C12N 1/15, A01N 63/00

(54) **Transgenic strains of trichoderma and their use in biocontrol**

(30) Priority: 22.09.2004 US 612028 P
(62) Divisional of application: 05808937.6
(71) Applicant: Bioworks, Inc., Fairport, NY 14450 (US)
(72) Inventor: Lorito, Matteo, 84080 Capezzano (IT); Mach, Robert, 1090 Vienna (AT); Zeilinger, Susanne, 1060 Vienna (AT); Woo, Sheridan, 84080 Capezzano (IT)
(74) Representative: Jones, Stephen Anthony

(57) **Abstract**

The present invention relates to a transgenic *Trichoderma* spp. having a recombinant nucleic acid molecule encoding a bioactive molecule. The present invention also relates to methods for controlling plant disease that involve applying a transgenic strain of *Trichoderma* spp. to plants or plants seeds, where the transgenic strain of *Trichoderma* spp. has a recombinant nucleic acid molecule encoding a bioactive molecule that is capable of controlling plant disease and conferring systemic disease resistance to the plant or a plant grown from the plant seed. The present invention also relates to a method of delivering a bioactive molecule to a plant or plant seed. The method involves providing a transgenic strain of *Trichoderma* spp. having a recombinant nucleic acid molecule encoding a bioactive molecule and applying the transgenic strain of *Trichoderma* spp. to the plant's roots or seeds.

SEQUENCE LISTING

<110> Lorito, Matteo
      Mach, Robert L.
      Zeilinger, Susanne
      Woo, Sheridan L.

<120> TRANSGENIC STRAINS OF TRICHODERMA AND THEIR USE IN
      BIOCONTROL

<130> 22027/1002

<140>
<141>

<150> 60/612,028
<151> 2004-09-22

<150> PCT/US2005/033762
<151> 2005-09-21

<160> 4

<170> PatentIn Ver. 2.1

<210> 1
<211> 1818
<212> DNA
<213> A. niger

<400> 1
atgcagactc tccttgtgag ctcgcttgtg gtctccctcg ctgcggccct gccacactac 60
atcaggagca atggcattga agccagcctc ctgactgatc ccaaggatgt ctccggccgc 120
acggtcgact acatcatcgc tggtggaggt ctgactggac tcaccaccgc tgctcgtctg 180
acggagaacc ccaacatcag tgtgctcgtc atcgaaagtg gctcctacga gtcggacaga 240
ggtcctatca ttgaggacct gaacgcctac ggcgacatct ttggcagcag tgtagaccac 300
gcctacgaga ccgtggagct cgctaccaac aatcaaaccg cgctgatccg ctccggaaat 360
ggtctcggtg gctctactct agtgaatggt ggcacctgga ctcgccccca caaggcacag 420
gttgactctt gggagactgt ctttggaaat gagggctgga actgggacaa tgtggccgcc 480
tactccctcc aggctgagcg tgctcgcgca ccaaatgcca aacagatcgc tgctggccac 540
tacttcaacg catcctgcca tggtgttaat ggtactgtcc atgccggacc ccgcgacacc 600
ggcgatgact attctcccat cgtcaaggct ctcatgagcg ctgtcgaaga ccggggcgtt 660
cccaccaaga aagacttcgg atgcggtgac ccccatggtg tgtccatgtt ccccaacacc 720
ttgcacgaag accaagtgcg ctccgatgcc gctcgcgaat ggctacttcc caactaccaa 780
cgtcccaacc tgcaagtcct gaccggacag tatgttggta aggtgctcct tagccagaac 840
ggcaccaccc ctcgtgccgt tggcgtggaa ttcggcaccc acaagggcaa cacccacaac 900
gtttacgcta agcacgaggt cctcctggcc gcgggctccg ctgtctctcc cacaatcctc 960
gaatattccg gtatcggaat gaagtccatc ctggagcccc ttggtatcga caccgtcgtt 1020
gacctgcccg tcggcttgaa cctgcaggac cagaccaccg ctaccgtccg ctcccgcatc 1080
acctctgctg gtgcaggaca gggacaggcc gcttggttcg ccaccttcaa cgagaccttt 1140
ggtgactatt ccgaaaaggc acacgagctg ctcaacacca agctggagca gtgggccgaa 1200
gaggccgtcg cccgtggcgg attccacaac accaccgcct tgctcatcca gtacgagaac 1260
taccgcgact ggattgtcaa ccacaacgtc gcgtactcgg aactcttcct cgacactgcc 1320
ggagtagcca gcttcgatgt gtgggacctt ctgcccttca cccgaggata cgttcacatc 1380
ctcgacaagg acccctacct tcaccacttc gcctacgacc ctcagtactt cctcaacgag 1440
ctggacctgc tcggtcaggc tgccgctact caactggccc gcaacatctc caactccggt 1500
gccatgcaga cctacttcgc tggggagact atccccggtg ataacctcgc gtatgatgcc 1560

gatttgagcg cctggactga gtacatcccg taccacttcc gtcctaacta ccatggcgtg 1620
ggtacttgct ccatgatgcc gaaggagatg ggcggtgttg ttgataatgc tgcccgtgtg 1680
tatggtgtgc agggactgcg tgtcattgat ggttctattc ctcctacgca aatgtcgtcc 1740
catgtcatga cggtgttcta tgccatggcg ctaaaaattt cggatgctat cttggaagat 1800
tatgcttcca tgcagtga 1818

<210> 2
<211> 605
<212> PRT
<213> A. niger

<400> 2
Met Gln Thr Leu Leu Val Ser Ser Leu Val Val Ser Leu Ala Ala Ala
1               5                   10                  15

Leu Pro His Tyr Ile Arg Ser Asn Gly Ile Glu Ala Ser Leu Leu Thr
            20                  25                  30

Asp Pro Lys Asp Val Ser Gly Arg Thr Val Asp Tyr Ile Ile Ala Gly
        35                  40                  45

Gly Gly Leu Thr Gly Leu Thr Thr Ala Ala Arg Leu Thr Glu Asn Pro
    50                  55                  60

Asn Ile Ser Val Leu Val Ile Glu Ser Gly Ser Tyr Glu Ser Asp Arg
65                  70                  75                  80

Gly Pro Ile Ile Glu Asp Leu Asn Ala Tyr Gly Asp Ile Phe Gly Ser
                85                  90                  95

Ser Val Asp His Ala Tyr Glu Thr Val Glu Leu Ala Thr Asn Asn Gln
            100                 105                 110

Thr Ala Leu Ile Arg Ser Gly Asn Gly Leu Gly Gly Ser Thr Leu Val
        115                 120                 125

Asn Gly Gly Thr Trp Thr Arg Pro His Lys Ala Gln Val Asp Ser Trp
    130                 135                 140

Glu Thr Val Phe Gly Asn Glu Gly Trp Asn Trp Asp Asn Val Ala Ala
145                 150                 155                 160

Tyr Ser Leu Gln Ala Glu Arg Ala Arg Ala Pro Asn Ala Lys Gln Ile
                165                 170                 175

Ala Ala Gly His Tyr Phe Asn Ala Ser Cys His Gly Val Asn Gly Thr
            180                 185                 190

Val His Ala Gly Pro Arg Asp Thr Gly Asp Asp Tyr Ser Pro Ile Val
        195                 200                 205

Lys Ala Leu Met Ser Ala Val Glu Asp Arg Gly Val Pro Thr Lys Lys
    210                 215                 220

Asp Phe Gly Cys Gly Asp Pro His Gly Val Ser Met Phe Pro Asn Thr
225                 230                 235                 240

Leu His Glu Asp Gln Val Arg Ser Asp Ala Ala Arg Glu Trp Leu Leu
                245                 250                 255

Pro Asn Tyr Gln Arg Pro Asn Leu Gln Val Leu Thr Gly Gln Tyr Val
            260                 265                 270

Gly Lys Val Leu Leu Ser Gln Asn Gly Thr Thr Pro Arg Ala Val Gly
        275                 280                 285

Val Glu Phe Gly Thr His Lys Gly Asn Thr His Asn Val Tyr Ala Lys
    290                 295                 300

His Glu Val Leu Leu Ala Ala Gly Ser Ala Val Ser Pro Thr Ile Leu
305                 310                 315                 320

Glu Tyr Ser Gly Ile Gly Met Lys Ser Ile Leu Glu Pro Leu Gly Ile
                325                 330                 335

Asp Thr Val Val Asp Leu Pro Val Gly Leu Asn Leu Gln Asp Gln Thr
            340                 345                 350

Thr Ala Thr Val Arg Ser Arg Ile Thr Ser Ala Gly Ala Gly Gln Gly
        355                 360                 365

Gln Ala Ala Trp Phe Ala Thr Phe Asn Glu Thr Phe Gly Asp Tyr Ser
    370                 375                 380

Glu Lys Ala His Glu Leu Leu Asn Thr Lys Leu Glu Gln Trp Ala Glu
385                 390                 395                 400

Glu Ala Val Ala Arg Gly Gly Phe His Asn Thr Thr Ala Leu Leu Ile
                405                 410                 415

Gln Tyr Glu Asn Tyr Arg Asp Trp Ile Val Asn His Asn Val Ala Tyr
            420                 425                 430

Ser Glu Leu Phe Leu Asp Thr Ala Gly Val Ala Ser Phe Asp Val Trp
        435                 440                 445

Asp Leu Leu Pro Phe Thr Arg Gly Tyr Val His Ile Leu Asp Lys Asp
    450                 455                 460

Pro Tyr Leu His His Phe Ala Tyr Asp Pro Gln Tyr Phe Leu Asn Glu
465                 470                 475                 480

Leu Asp Leu Leu Gly Gln Ala Ala Ala Thr Gln Leu Ala Arg Asn Ile
                485                 490                 495

Ser Asn Ser Gly Ala Met Gln Thr Tyr Phe Ala Gly Glu Thr Ile Pro
            500                 505                 510

Gly Asp Asn Leu Ala Tyr Asp Ala Asp Leu Ser Ala Trp Thr Glu Tyr
        515                 520                 525

Ile Pro Tyr His Phe Arg Pro Asn Tyr His Gly Val Gly Thr Cys Ser
    530                 535                 540

Met Met Pro Lys Glu Met Gly Gly Val Val Asp Asn Ala Ala Arg Val
545                 550                 555                 560

Tyr Gly Val Gln Gly Leu Arg Val Ile Asp Gly Ser Ile Pro Pro Thr
                565                 570                 575

Gln Met Ser Ser His Val Met Thr Val Phe Tyr Ala Met Ala Leu Lys
            580                 585                 590

Ile Ser Asp Ala Ile Leu Glu Asp Tyr Ala Ser Met Gln
        595                 600                 605


<210> 3
<211> 405
<212> DNA
<213> Cladosporium fulvum

<400> 3
atgcactaca caaccctcct cctaagcact ctcctagtcg gcacagccct cgcccaaccc 60
acaaatcctc ctgcgaagac tcccaagaag gcccccaaaa ctcaaccata caacccatgc 120
aagccccaag aagtcatcga caccaagtgt atgggtccca aggattgtct ctacccgaac 180
cccgacagtt gtacaaccta catacagtgt gtaccgctcg acgaagttgg caatgcgaag 240
cctgtggtta agccatgtcc aaaaggactg cagtggaacg ataacgttgg caagaagtgg 300
tgcgactatc caaacctgag tacgtgtccg gtaaagacgc cgcaaccgaa gccgaagaag 360
ggaggtgtcg gagggaagaa ggcgtcggtt ggacatcctg gctat                405


<210> 4
<211> 191
<212> DNA
<213> Cladosporium fulvum

<400> 4
atgaagcttt ccctccttag cgtagagctt gctctcctaa ttgctactac tctcccactt 60
tgctgggcag ctgccctccc tgtaggattg ggagtcgggc tagactactg taactcaagt 120
tgtactaggg ccttcgactg tttgggtcaa tgtggcagat gcgactttca taagctacaa 180
tgtgtccact a                                                      191

## Description

This application claims benefit of U.S. Provisional Patent Application Serial No. 60/612,028, filed September 22, 2004, which is hereby incorporated by reference in its entirety.

### FIELD OF THE INVENTION

The present invention relates to a transgenic *Trichoderma* spp. having a recombinant nucleic acid molecule encoding a bioactive molecule and methods of using such transgenic *Trichoderma* spp. for controlling plant disease and delivering bioactive molecules to plants and plant seeds.

### BACKGROUND OF THE INVENTION

Fungi in the genus *Trichoderma* are being used in increasingly large amounts for control of plant diseases. These fungi recently have been shown to be avirulent plant symbionts. *Trichoderma* spp. are highly interactive in root, soil, and foliar environments. The most useful of these strains colonize the outer surface of plant roots and produce various signaling molecules. This ability to colonize plant roots and to deliver bioactive plant molecules *in vivo* makes these fungi highly effective systems for the delivery of bioactive molecules. It has been known for many years that *Trichoderma* produce a wide range of antibiotic substances (Sivasithamparam et al., "Secondary Metabolism in Trichoderma and Gliocladium," in Kubicek et al., Trichoderma and Gliocladium, Vol 1, London: Taylor and Francis, pp. 139-191 (1998)) and that they are parasitic on other fungi. They also compete with other microorganisms, for example, for key exudates from seeds that stimulate germination of propagules of plant pathogenic fungi in soil (Howell, C. R., "Cotton Seedling Preemergence Damping-Off Incited by Rhizopus oryzae and Pythium spp. and its Biological Control with Trichoderma spp.," Phytopathology 92:177-180 (2002)). More generally, they compete with soil microorganisms for nutrients or space (Elad, Y., "Mechanisms Involved in the Biological Control of Botrytis Cinerea Incited Diseases," Eur J Plant Pathol 102:719-732 (1996)). In addition, they inhibit or degrade pectinases and other enzymes that are essential for plant pathogenic fungi such as *Botrytis cinerea* to penetrate leaf surfaces (Zimand et al., "Effect of Trichoderma harzianum on Botrytis cinerea Pathogenicity," Phytopathology 86:1255-1260 (1996)). These direct effects upon other fungi are complex and remarkable and, until very recently, were considered to be the bases for how *Trichoderma* spp. exert beneficial effects on plant growth and development. The research on these topics has generated a large body of knowledge including isolation and cloning of a range of genes that encode for proteins and metabolites, some of which have antimicrobial activity. There are several recent reviews on these compounds and mechanisms of action (Benitez et al., "Glucanolytic and Other Enzymes and Their Control. In Trichoderma and Gliocladium, in Harman et al., eds., Vol 2, London: Taylor and Francis, pp. 101-127 (1998); Chet et al., "Mycoparasitism and Lytic Enzymes," in Harman et al., Trichoderma and Gliocladium, Vol 2, London: Taylor and Francis, pp. 153-172 (1998); Howell, C. R., "Mechanisms Employed by Trichoderma Species in the Biological Control of Plant Diseases: The History and Evolution of Current Concepts," Plant Dis 87:4-10 (2003); Lorito, M., "Chitinolytic Enzymes and Their Genes. In Trichoderma and Gliocladium, Vol 2, pp. 73-99. Edited by G. E. Harman & C. P. Kubicek. London: Taylor and Francis. (1998)). This research has produced several useful findings including the use of genes encoding fungitoxic cell wall degrading enzymes to produce transgenic plants resistant to diseases (Bolar et al., "Synergistic Activity of Endochitinase and Exochitinase from Trichoderma atroviride (T. harzianum) Against the Pathogenic Fungus (Venturia inaequalis) in Transgenic Apple Plants," Trans Res 10:533-543 (2001); Lorito et al., "Microbial Genes Expressed in Transgenic Plants to Improve Disease Resistance," Journal of Plant Pathology 81:73-88 (1999)) and the discovery of enzymes useful in bioprocessing of chitin (Donzelli et al., "Enhanced Enzymatic Hydrolysis of Langostino Shell Chitin With Mixtures of Enzymes from Bacterial and Fungal Sources," Carboh Res 338:1823-1833 (2003)). However, it is becoming increasingly clear that the understanding of the mechanisms of biocontrol has been incomplete. In addition to the ability of *Trichoderma* spp. to directly attack or inhibit growth of plant pathogens, recent discoveries indicate that they can also induce systemic and localized resistance to a variety of plant pathogens. These new findings are dramatically changing knowledge of the mechanisms of action and uses of these fungi.

Moreover, certain strains of *Trichoderma* have substantial influence upon plant growth and development. Enhancement of plant growth has been known for many years and can occur in either axenic systems (Lindsey et al., "Effect of Certain Fungi on Dwarf Tomatoes Grown Under Gnotobiotic Conditions," Phytopathology 57:1262-1263 (1967); Yedidia et al., "Effect of Trichoderma harzianum on Microelement Concentrations and Increased Growth of Cucumber Plants," Plant Soil 235:235-242 (2001)) or in natural field soils (Chang et al., "Increased Growth of Plants in the Presence of the Biological Control Agent Trichoderma harzianum," Plant Dis 70:145-148 (1986); Harman, G. E., "Myths and Dogmas of Biocontrol. Changes in Perceptions Derived from Research on Trichoderma harzianum T-22," Plant Dis 84:377-393 (2000)). The direct effects of these fungi on plant growth and development are critically important for agricultural uses and also for understanding the roles of *Trichoderma* in natural and managed ecosystems.

Localized and systemic induced resistance occurs in all or most plants in response to attack by pathogenic microbes, physical damage by insects or other factors, treatment with various chemical inducers or the presence of nonpathogenic rhizobacteria (Kuc, J., "Concepts and Direction of Induced Systemic Resistance in Plants and its Application," Eur J Plant Pathol 107:7-12 (2001); Oostendorp et al., "Induced Resistance in Plants by Chemicals," Eur J Plant Pathol 107:19-28 (2001)). Much progress has been made in elucidating the pathways of this resistance. In many cases, salicylic acid or jasmonic acid, together with ethylene or nitrous oxide, induce a cascade of events leading to the production of a variety of metabolites and proteins with diverse functions (Hammerschmidt et al., "Inducing Resistance: A Summary of Papers Presented at the First International Symposium on Induced Resistance to Plant Diseases," Corfu, May 2000, Eur J Plant Pathol 107:1-6 (2001); van Loon et al., "Systemic Resistance Induced by Rhizosphere Bacteria," Annu Rev Phytopathol 36:453-483 (1998)). Different pathways are induced by different challenges, even though there seems to be cross-talk or competition between the pathways (Bostock et al., "Signal Interactions in Induced Resistance to Pathogens and Insect Herbivores," Eur J Plant Pathol 107:103-111 (2001)).

In recent years, substantial advances have been made in identifying the induced systemic resistance pathway activated by rhizobacteria, which is the closest analog of induced resistance by *Trichoderma*. The rhizobacteria-induced systemic resistance (RISR) pathway phenotypically resembles systemic acquired resistance (SAR) systems in plants. However, RISR differs in the fact that root colonization by rhizobacteria does not result in the detectable expression of pathogenesis-related proteins, and root colonization by at least some bacterial strains does not induce accumulation of salicylic acid in the plant (Bakker et al., "Understanding the Involvement of Rhizobacteria-Mediated Induction of Systemic Resistance in Involvement of Rhizobacteria-Mediated Induction of Systemic Biocontrol of Plant Diseases," Can J Plant Pathol 25:5-9 (2003)). Instead, plants are potentiated to react rapidly to pathogen attack. In *Arabidopsis,* RISR requires functional plant responses to jasmonic acid and ethylene, and may increase sensitivity to them, and is, like SAR, dependent upon the transcription factor NPR1 (Pieterse et al., "Rhizobacteria-Mediated Induced Systemic Resistance: Triggering, Signalling and Expression," Eur J Plant Pathol 107:51-61 (2001)). The abilities of rhizobacteria to induce systemic resistance have long been known (Kloepper et al., "Plant Growth Promoting Rhizobacteria as Inducers of Systemic Acquired Resistance," in Lumsden et al., eds., Pest Management: Biologically Based Technologies, Washington, D. C., pp. 10-20 (1993); Pieterse et al., "Rhizobacteria-Mediated Induced Systemic Resistance: Triggering, Signalling and Expression," Eur J Plant Pathol 107:51-61 (2001)).

*Talaromyces flavus* has been suggested as a biocontrol agent against the plant pathogens *Verticillium dahliae* (Marois et al., "Biological Control of Verticillium Wilt of Eggplant in the Field," In Plant Disease, pp. 1166-1168 (1982)), *Sclerotinia sclerotiorum* (McLaren et al., "Hyperparasitism of Sclerotinia sclerotiorum by Talaromyces flavus," J Plant Pathol 8:43-48 (1986)) and *Rhizoctonia solani* (Boosalis, M. G., "Effect of Soil Temperature and Green-Manure Amendment of Unsterilized Soil on Parasitism of Rhizoctonia solani by Penicillum vermiculatum and Trichoderma sp.," Phytopathology 46:473-478 (1956)). *In vitro* experiments performed with culture filtrates of *T. flavus* grown on glucose suggest that glucose oxidase is responsible for most of the growth inhibition of *V*. *dahliae* microsclerotia and hyphae (Murray et al., "Isolation of the Glucose Oxidase Gene from Talaromyces flavus and Characterization of its Role in the Biocontrol of Verticillium dahliae," Curr Genet 32:367-375 (1997); Stosz et al., "In vitro Analysis of the Role of Glucose Oxidase from Talaromyces flavus in Biocontrol of the Plant Pathogen Verticillium dahliae," Appl Environ Microbiol 62:3183-3186 (1996)). A glucose oxidase deficient strain of *T. flavus* also failed to antagonize *Verticillium* wilt of eggplant in greenhouse experiments (Brunner et al., "The Nag1 N-acetylglucosaminidase of Trichoderma atroviride is Essential for Chitinase Induction by Chitin and of Major Relevance to Biocontrol," Curr Genet 43:289-295 (2003)). Glucose oxidase catalyzes the oxygen-dependent oxidation of D-glucose to D-glucono-1,5-lactone and hydrogen peroxide (H₂O₂). Glucose oxidase, glucose, and gluconate (which is spontaneously formed from D-glucono-1,5-lactone in aqueous solutions) do not inhibit *V*. *dahlia* when used individually (Kim et al., "Glucose Oxidase as the Antifungal Principle of Talaron from Talaromyces flavus," Can J Microbiol 36:760-764 (1993)) but low concentrations of H₂O₂ significantly inhibit the growth of *Pythium ultimum, P. aphanidermatum, R. solani,* and *V. dahliae*. Therefore, the antifungal effect of the glucose oxidase system is due to increased levels of H₂O₂ (Inglis et al., "Comparative Degradation of Oomycete, Ascomycete, and Basidiomycete Cell Walls by Mycoparasitic Biocontrol Fungi," Can J Microbiol 48:60-70 (2002)).

Production of hydrogen peroxide and reactive oxygen species is associated with plant response to pathogen attack and involved in the so-called oxidative burst. These compounds are considered to play multiple roles in plant defense, such as triggering of the hypersensitivity reaction (HR), exerting direct antimicrobial activity, diffusing the signal for activation of defense genes, and reinforcing the plant cell wall (Mourgues et al., Strategies to Improve Plant Resistance to Bacterial Diseases Through Genetic Engineering," Trends Biotechnol 16(5):203-10 (1998)). For this reasons, the production of H₂O₂ has been considered as a target for genetic improvement of plant pathogen resistance by modifying, for instance, either SOD or catalase activity (Heinen et al., "Increased Resistance to Oxidative Stress in Transgenic Plants That Overexpress Chloroplastic Cu/Zn Superoxidase Dismutase," Proc Natl Acad Sci USA 90(4):1629-1633 (1993), H. Sanderman, Jr., "Active oxygen Species as Mediators of Plant Immunity; Three Case Studies," J. Biol Chem 381:649-653 (2000)). In addition, a fungal glucose-oxidase encoding gene from *A. niger* was expressed in potato by Wu et al., "Disease Resistance Conferred by Expression of a Gene Encoding H2O2-Generating Glucose Oxidase in Transgenic Potato Plants," Plant Cell 7(9):1357-68 (1995) to increase the level of H₂O₂ in transgenic tubers and leaves following bacterial infection. The progeny exhibited an improved resistance to both bacterial (*Erwinia amilovora* subsp. *carotovora*) and fungal (*Phytophthora infestans*) pathogens, while the addition of catalase counteracted the effect of the transgene. This work indicated that microbial enzymes capable of generating active oxygen species may confer resistance to a broad-spectrum of plant pathogens. Several species of *Trichoderma* are more resistant to the products of glucose oxidase activity than many plant pathogens (Kim et al., "Glucose Oxidase as the Antifungal Principle of Talaron from Talaromyces flavus," Can J Microbiol 36:760-764 (1993)), even though an orthologue of glucose oxidase has not been found so far in this fungus (Mach et al., "Expression of Two Major Chitinase Genes of Trichoderma atroviride (T. harzianum P1) is Triggered by Different Regulatory Signals," Appl Environ Microbiol 65:1858-1863 (1999)).

Although *Trichoderma* is clearly an important biocontrol agent, *Trichoderma* alone does not provide the broad-spectrum of microbial and fungal disease resistance conferred by microbial enzymes capable of generating active oxygen species such as H₂O₂. It would be highly useful to have the broad-spectrum anti-pathogenic capacity of a bioactive glucose oxidase producing, H₂O₂-generating bioagent in combination with an agent having the ability of *Trichoderma* to colonize plant roots and deliver bioactive molecules *in vivo.*

The present invention is directed to overcoming these and other deficiencies in the art.

### SUMMARY OF THE INVENTION

The present invention relates to a transgenic *Trichoderma* spp. having a recombinant nucleic acid molecule encoding a bioactive molecule, where the bioactive molecule is selected from the group consisting of a plant chitinase, a glucanase, a chitosanase, an endochitinase, an osmotin, a ribosome inactivating protein, a trichodiene sintasi, a stilbene sintasi, a killer toxin, a barnase, a ribonuclease, choleric toxin subunit A, a *Bacillus thuringiensis* toxin, an avirulence factor, a virulence factor, a β-cryptogein, a protonic pump, a pectate lyase, an oligogalacturonide lyase, a tabtoxin resistance protein, an ornitine carbamoyltransferase, a shiva-1, an attacinE, a lysozyme, a lactoferrin, a tachiplesin, resistance protein *Xa21*, a tionin, and a bacterial opsin.

The present invention also relates to a method of controlling plant disease. This involves applying a transgenic strain of *Trichoderma* spp. to a plant or plant seed, where the transgenic strain of *Trichoderma* spp. has a recombinant nucleic acid molecule encoding a bioactive molecule capable of controlling plant disease. The applying is carried out under conditions effective to control plant disease in the plant or a plant grown from the plant seed.

The present invention also relates to a method of delivering a bioactive molecule to a plant or plant seed. The method involves providing a transgenic strain of *Trichoderma* spp., where the transgenic strain of *Trichoderma* spp. comprises a recombinant nucleic acid molecule encoding a bioactive molecule, and applying the transgenic strain of *Trichoderma* spp. to a plant or plant seed. Application is carried out under conditions effective to deliver the bioactive molecule to the plant or plant seed.

The present invention provides a highly effective biocontrol agent and delivery system, having an improved ability to inhibit disease in plants and induce systemic resistance to diseases in plants caused by phytopathogens. Thus, the present invention provides a biologic alternative to the use of chemicals which may be highly attractive to commercial agriculture in instances where the availability of chemical pesticides have been lost to regulatory action or pest resistance, and in which there are no adequate chemical replacements.

### BRIEF DESCRIPTION OF THE DRAWINGS

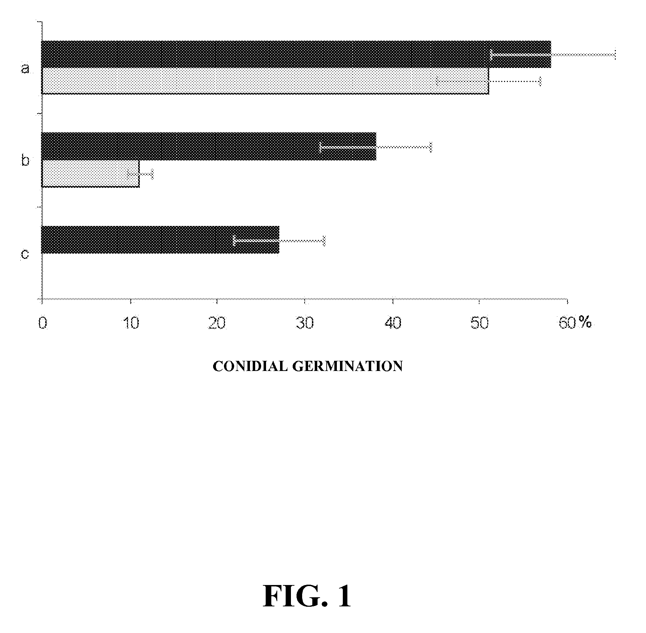

Figure 1 is a graph showing the percentage of conidia (relative to a water control) of *B. cinerea* conidia that germinated in the presence of filtrates obtained from *T. atroviride* strain P1 (black bars) or SJ3 4 (gray bars) under variance culture conditions. Shown are germination results (a) in the presence of glucose (low glucose oxidase production) or (b) in the presence of chitin as the carbon source; (c) is the germination percentage in P1 culture filtrates augmented with 8 mM H₂O₂.

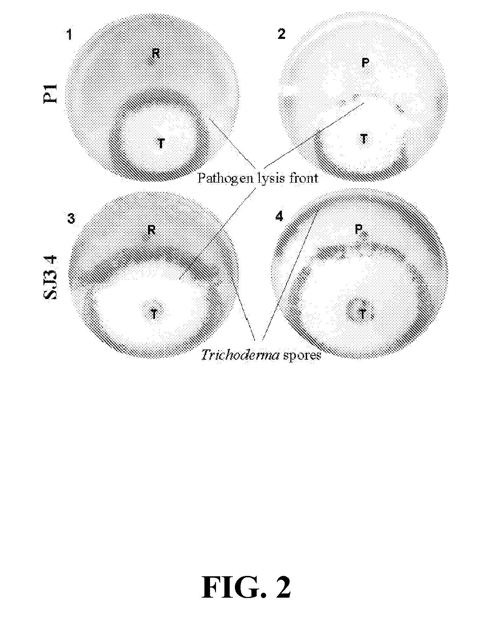

Figure 2 is a photograph showing the results of confrontation assays on potato dextrose agar ("PDA"), measuring direct parasitism of *Trichoderma* strains against target fungi. *Trichoderma* wild-type strain (P1, plates 1 and 2) versus the transgenic strain SJ3 4 (plates 3 and 4); targets: R *=R. solani*; P *= P ultimum.*

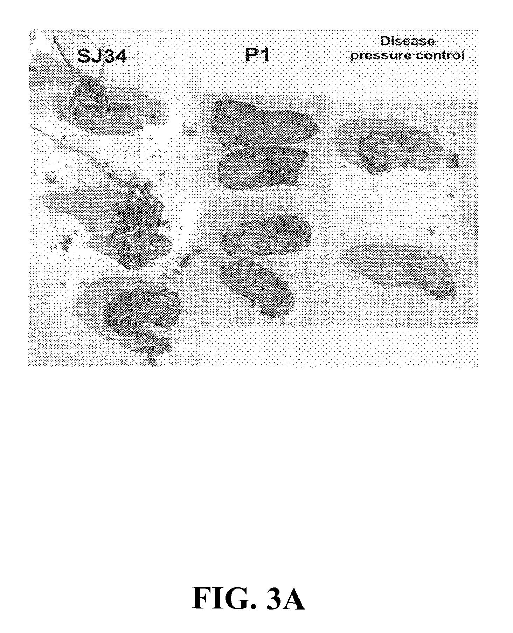

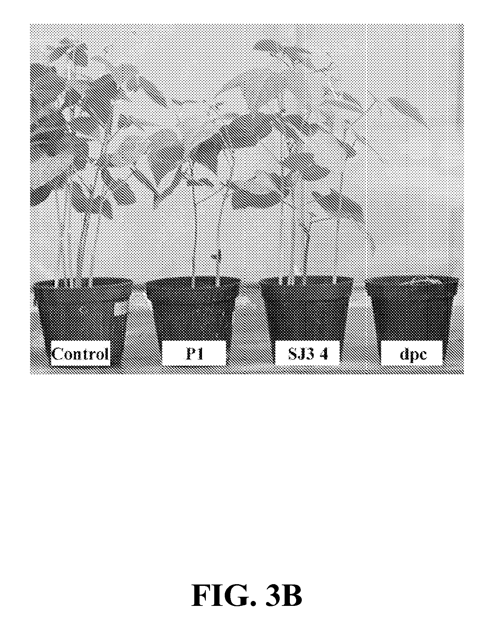

Figures 3A-B are photographs showing the results of *in planta* biocontrol assays conducted on bean plants. Figure 3A shows the effect of the gox transformation on seed germination with no pathogen added. Figure 3B is a photograph of plants infected with *R. solani* following seed treatment with wild type *Trichoderma* or transgenic gox-containing *Trichoderma*. Control = untreated bean plants; P1 = T. atroviride wild-type strain; SJ3 4 = transgenic gox-containing *Trichoderma*; dpc (disease pressure control) = R. solani only.

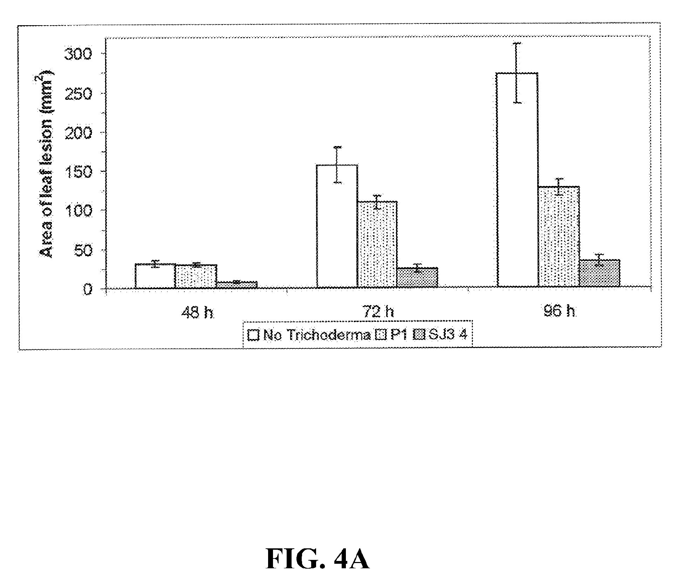

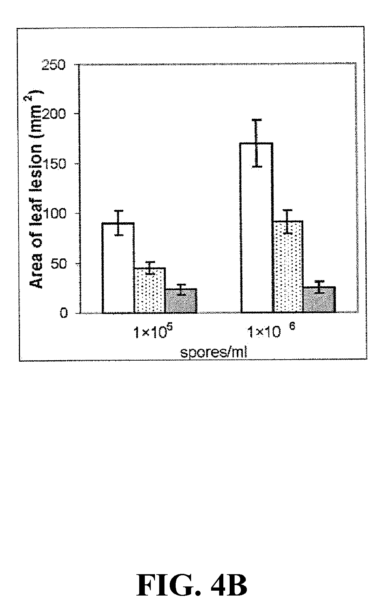

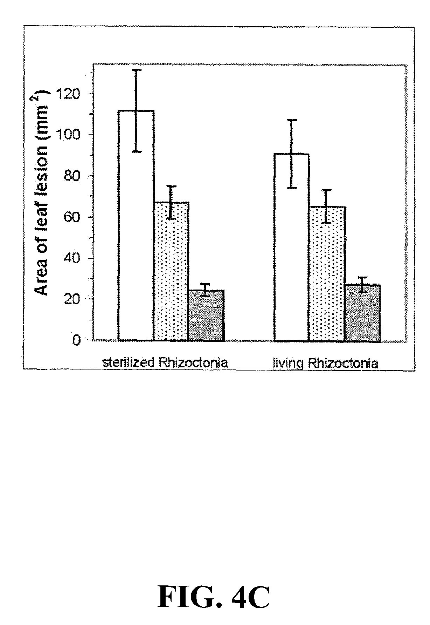

Figures 4A-C are graphs showing the results of a biocontrol experiment testing the ability of SJ3 4 to induce systemic resistance against foliar disease. The leaves of plants grown from seeds treated with *T. atroviride* strain P1, transgenic SJ3 4, or no *Trichoderma*, were inoculated with a plant pathogen. Disease development is measured as area of leaf lesion occurring in the inoculated plants. Figure 4A shows foliar disease development at different times after inoculations with *B. cinerea.* Figure 4B shows results after inoculations with different *B. cinerea* spore concentrations. Figure 4C shows areas of foliar disease caused by *B. cinerea* 72 hr after inoculation when plants were grown in soil infested with autoclaved or live mycelia of *Rhizoctonia solani.*

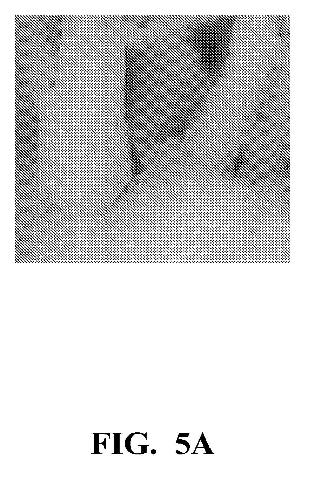

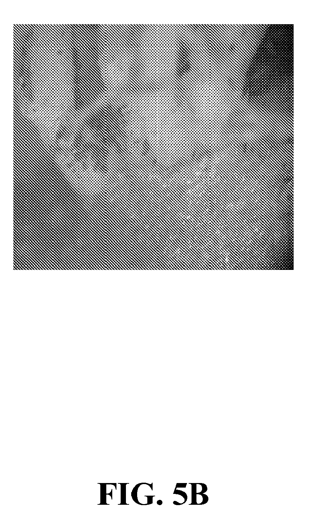

Figures 5A-B are photographs showing results of treatment with a transgenic *T. atroviride* strain P1 expressing the avirulence gene *avr4*. Figure 5A shows the roots of Cf4-containing tomato plants treated with wildtype *T*. *atroviride* strain P1. Figure 5B shows plant roots treated with *T. atroviride* strain P1 transformed with the avirulence gene, *avr4.*

### DETAILED DESCRIPTION OF THE INVENTION

The present invention relates to a transgenic *Trichoderma* spp. having a recombinant nucleic acid molecule encoding a bioactive molecule. The bioactive molecule is selected from the group consisting of a plant chitinase, a glucanase, a chitosanase, an endochitinase, an osmotin, a ribosome inactivating protein, a trichodiene sintasi, a stilbene sintasi, a killer toxin, a barnase, a ribonuclease, choleric toxin subunit A, a *Bacillus thuringiensis* toxin, an avirulence factor, a virulence factor, a β-cryptogein, a protonic pump, a pectate lyase, an oligogalacturonide lyase, a tabtoxin resistance protein, an ornitine carbamoyltransferase, a shiva-1, an attacinE, a lysozyme, a lactoferrin, a tachiplesin, resistance protein *Xa21*, a tionin, and a bacterial opsin.

Exemplary delivery organisms suitable for this and all other aspects of the present invention are fungi in the genus *Trichoderma* (U.S. Patent No. 5,260,213 to Harman et al., which is hereby incorporated by reference in its entirety), including *Trichoderma harzianum*; the protoplast fusion progeny of *Trichoderma harzianum* 1295-22, known as "T-22", (ATCC 20847) (U.S. Patent No. 5,260,213 to Harman et al.; Harman, G. E., "The Dogmas and Myths of Biocontrol. Changes in Perceptions Based on Research with Trichoderma harzianum T-22," Plant Dis. 84, 377-393 (2000), which are hereby incorporated by reference in their entirety), and T-22™ (BioWorks, Inc., Geneva, NY); and *T*. *virens*, formerly classified as *Gliocladium virens* (U.S. Patent No. 5,165,928 to Smith et al., which is hereby incorporated by reference in its entirety). Any natural, mutant, or fused strain of *Trichoderma* shown to be rhizosphere competent is also suitable for all aspects of the present invention.

*Trichoderma* are organisms with strong abilities to colonize roots. This ability is known as rhizosphere competence, which is used herein to describe those organisms capable of colonizing the root surface or the surface plus surrounding soil volume (rhizoplane and rhizosphere, respectively), when applied as a seed or other point source at the time of planting in absence of bulk flow of water. Thus, the agents of the present invention have the physiological and genetic ability to proliferate the root as it develops. Rhizosphere competence is not an absolute term, and degrees of this ability may occur among strains (Harman, G. E., "The Development and Benefits of Rhizosphere Competent Fungi for Biological Control of Plant Pathogens," J. Plant Nutrition 15:835-843 (1992); U.S. Patent Nos. 4,996,157 and 5,165,928 to Smith, which are hereby incorporated by reference in their entirety). Other organisms, including those in the genera *Bacillus*, *Pseudomonas,* and *Burkholderia* also possess good root competence (Brannen et al., "Kodiak: A Successful Biological-Control Product for Suppression of Soil-Borne Plant Pathogens of Cotton," J. Industr. Microbiol. Biotechnol. 19 (1997) 169-171 (1997); Kloepper et al. "Plant Growth Promoting Rhizobacteria As Inducers of Systemic Acquired Resistance," In: Lumsden, R. D. and Vaughn, J. L. (ed.): Pest Management: Biologically Based Technologies. Washington, D. C., pp. 10-20 (1993), which are hereby incorporated by reference in their entirety). Procedures for measuring rhizosphere competence are well-known in the art (Harman et al., "Combining Effective Strains of Trichoderma harzianum and Solid Matrix Priming to Improve Biological Seed Treatments," Plant Dis. 73:631-637 (1989); Harman, G. E., "The Dogmas and Myths of Biocontrol. Changes in Perceptions Based on Research with Trichoderma harzianum T-22," Plant Dis. 84, 377-393 (2000); Kloepper et al., "A Review of Issues Related to Measuring Colonization of Plant Roots by Bacteria," Can J. Microbiol. 38, 1219-1232 (1992), which are hereby incorporated by reference in their entirety).

Suitable nucleic acid molecules for use in this aspect and all other aspects of the present invention are any nucleic acid molecules isolated from any source, including, but not limited to, bacteria, fungi, and plants that express bioactive molecules capable of controlling plant disease and conferring systemic disease resistance to plants. In one aspect of the present invention, the nucleic acid molecule is a heterologous nucleic acid molecule (i.e., foreign to *Trichoderma* spp.). Alternatively, the nucleic acid molecule inserted into the selected expression system may be homologous (i.e., native to *Trichoderma* spp.). In some aspects of the present invention the bioactive molecule is a glucose oxidase (gox) protein or polypeptide, and the nucleic acid molecule is any nucleic acid molecule encoding glucose oxidase (*gox*). Suitable gox nucleic acid molecules may be derived from any of the microbial species that contain a *gox* gene, including, but not limited to, *T*. *flavus* (Llwellyn et al., "Isolation of Glucose Oxidase Gene from Talaromyces flavus and Characterisation of it's Role in the Biocontrol of Verticillium dahliae," Curr Genet 32(5):367-375 (1997); Fravel et al., "In vitro Analysis of the Role of Glucose Oxidase from Talaromyces flavus in the Biocontrol of Verticillium dahliae," Appl Environ Microbiol 62(9):3183-86 (1996); Kim et al., "Glucose Oxidase as the Antifungal Principle of Talaron from Talaromyces flavus," Can J. Microbiol 36(11):760-764 (1990), which are hereby incorporated by reference in their entirety) and *A. niger* (e.g., ATCC. 9029; Mach et al., "Expression of Two Major Chitinase Genes of Trichoderma atroviride (T. harzianum P1) is Triggered by Different Regulatory Signals," Appl Environ Microbiol 65:1858-1863 (1999); Kriechbaum et al., "Cloning and DNA Sequence analysis of the Glucose Oxidase Gene from Aspergillus niger NRRL-3," FEBS Letters 255(1):63-66 (1989), which are hereby incorporated by reference in their entirety).

A preferred nucleic acid molecule for the present invention is a nucleic acid molecule derived from *A*. *niger*, having SEQ ID NO:1, as follows: 
atgcagactc tccttgtgag ctcgcttgtg gtctccctcg ctgcggccct gccacactac 60
atcaggagca atggcattga agccagcctc ctgactgatc ccaaggatgt ctccggccgc 120
acggtcgact acatcatcgc tggtggaggt ctgactggac tcaccaccgc tgctcgtctg 180
acggagaacc ccaacatcag tgtgctcgtc atcgaaagtg gctcctacga gtcggacaga 240
ggtcctatca ttgaggacct gaacgcctac ggcgacatct ttggcagcag tgtagaccac 300
gcctacgaga ccgtggagct cgctaccaac aatcaaaccg cgctgatccg ctccggaaat 360
ggtctcggtg gctctactct agtgaatggt ggcacctgga ctcgccccca caaggcacag 420
gttgactctt gggagactgt ctttggaaat gagggctgga actgggacaa tgtggccgcc 480
tactccctcc aggctgagcg tgctcgcgca ccaaatgcca aacagatcgc tgctggccac 540
tacttcaacg catcctgcca tggtgttaat ggtactgtcc atgccggacc ccgcgacacc 600
ggcgatgact attctcccat cgtcaaggct ctcatgagcg ctgtcgaaga ccggggcgtt 660
cccaccaaga aagacttcgg atgcggtgac ccccatggtg tgtccatgtt ccccaacacc 720
ttgcacgaag accaagtgcg ctccgatgcc gctcgcgaat ggctacttcc caactaccaa 780
cgtcccaacc tgcaagtcct gaccggacag tatgttggta aggtgctcct tagccagaac 840
ggcaccaccc ctcgtgccgt tggcgtggaa ttcggcaccc acaagggcaa cacccacaac 900
gtttacgcta agcacgaggt cctcctggcc gcgggctccg ctgtctctcc cacaatcctc 960
gaatattccg gtatcggaat gaagtccatc ctggagcccc ttggtatcga caccgtcgtt 1020
gacctgcccg tcggcttgaa cctgcaggac cagaccaccg ctaccgtccg ctcccgcatc 1080
acctctgctg gtgcaggaca gggacaggcc gcttggttcg ccaccttcaa cgagaccttt 1140
ggtgactatt ccgaaaaggc acacgagctg ctcaacacca agctggagca gtgggccgaa 1200
gaggccgtcg cccgtggcgg attccacaac accaccgcct tgctcatcca gtacgagaac 1260
taccgcgact ggattgtcaa ccacaacgtc gcgtactcgg aactcttcct cgacactgcc 1320
ggagtagcca gcttcgatgt gtgggacctt ctgcccttca cccgaggata cgttcacatc 1380
ctcgacaagg acccctacct tcaccacttc gcctacgacc ctcagtactt cctcaacgag 1440
ctggacctgc tcggtcaggc tgccgctact caactggccc gcaacatctc caactccggt 1500
gccatgcaga cctacttcgc tggggagact atccccggtg ataacctcgc gtatgatgcc 1560
gatttgagcg cctggactga gtacatcccg taccacttcc gtcctaacta ccatggcgtg 1620
ggtacttgct ccatgatgcc gaaggagatg ggcggtgttg ttgataatgc tgcccgtgtg 1680
tatggtgtgc agggactgcg tgtcattgat ggttctattc ctcctacgca aatgtcgtcc 1740
catgtcatga cggtgttcta tgccatggcg ctaaaaattt cggatgctat cttggaagat 1800
tatgcttcca tgcagtga 1818
 This nucleic acid molecule encodes a gox protein having the amino acid of SEQ ID NO:2, as follows:

Met Gln Thr Leu Leu Val Ser Ser Leu Val Val Ser Leu Ala Ala Ala
1               5                   10                  15

Leu Pro His Tyr Ile Arg Ser Asn Gly Ile Glu Ala Ser Leu Leu Thr
            20                  25                  30

Asp Pro Lys Asp Val Ser Gly Arg Thr Val Asp Tyr Ile Ile Ala Gly
        35                  40                  45

Gly Gly Leu Thr Gly Leu Thr Thr Ala Ala Arg Leu Thr Glu Asn Pro
    50                  55                  60

Asn Ile Ser Val Leu Val Ile Glu Ser Gly Ser Tyr Glu Ser Asp Arg
65                  70                  75                  80

Gly Pro Ile Ile Glu Asp Leu Asn Ala Tyr Gly Asp Ile Phe Gly Ser
                85                  90                  95

Ser Val Asp His Ala Tyr Glu Thr Val Glu Leu Ala Thr Asn Asn Gln
            100                 105                 110

Thr Ala Leu Ile Arg Ser Gly Asn Gly Leu Gly Gly Ser Thr Leu Val
        115                 120                 125

Asn Gly Gly Thr Trp Thr Arg Pro His Lys Ala Gln Val Asp Ser Trp
    130                 135                 140

Glu Thr Val Phe Gly Asn Glu Gly Trp Asn Trp Asp Asn Val Ala Ala
145                 150                 155                 160

Tyr Ser Leu Gln Ala Glu Arg Ala Arg Ala Pro Asn Ala Lys Gln Ile
                165                 170                 175

Ala Ala Gly His Tyr Phe Asn Ala Ser Cys His Gly Val Asn Gly Thr
            180                 185                 190

Val His Ala Gly Pro Arg Asp Thr Gly Asp Asp Tyr Ser Pro Ile Val
        195                 200                 205

Lys Ala Leu Met Ser Ala Val Glu Asp Arg Gly Val Pro Thr Lys Lys
    210                 215                 220

Asp Phe Gly Cys Gly Asp Pro His Gly Val Ser Met Phe Pro Asn Thr
225                 230                 235                 240

Leu His Glu Asp Gln Val Arg Ser Asp Ala Ala Arg Glu Trp Leu Leu
                245                 250                 255

Pro Asn Tyr Gln Arg Pro Asn Leu Gln Val Leu Thr Gly Gln Tyr Val
            260                 265                 270

Gly Lys Val Leu Leu Ser Gln Asn Gly Thr Thr Pro Arg Ala Val Gly
        275                 280                 285

Val Glu Phe Gly Thr His Lys Gly Asn Thr His Asn Val Tyr Ala Lys
    290                 295                 300

His Glu Val Leu Leu Ala Ala Gly Ser Ala Val Ser Pro Thr Ile Leu
305                 310                 315                 320

Glu Tyr Ser Gly Ile Gly Met Lys Ser Ile Leu Glu Pro Leu Gly Ile
                325                 330                 335

Asp Thr Val Val Asp Leu Pro Val Gly Leu Asn Leu Gln Asp Gln Thr
            340                 345                 350

Thr Ala Thr Val Arg Ser Arg Ile Thr Ser Ala Gly Ala Gly Gln Gly
        355                 360                 365

Gln Ala Ala Trp Phe Ala Thr Phe Asn Glu Thr Phe Gly Asp Tyr Ser
    370                 375                 380

Glu Lys Ala His Glu Leu Leu Asn Thr Lys Leu Glu Gln Trp Ala Glu
385                 390                 395                 400

Glu Ala Val Ala Arg Gly Gly Phe His Asn Thr Thr Ala Leu Leu Ile
                405                 410                 415

Gln Tyr Glu Asn Tyr Arg Asp Trp Ile Val Asn His Asn Val Ala Tyr
            420                 425                 430

Ser Glu Leu Phe Leu Asp Thr Ala Gly Val Ala Ser Phe Asp Val Trp
        435                 440                 445

Asp Leu Leu Pro Phe Thr Arg Gly Tyr Val His Ile Leu Asp Lys Asp
    450                 455                 460

Pro Tyr Leu His His Phe Ala Tyr Asp Pro Gln Tyr Phe Leu Asn Glu
465                 470                 475                 480

Leu Asp Leu Leu Gly Gln Ala Ala Ala Thr Gln Leu Ala Arg Asn Ile
                485                 490                 495

Ser Asn Ser Gly Ala Met Gln Thr Tyr Phe Ala Gly Glu Thr Ile Pro
            500                 505                 510

Gly Asp Asn Leu Ala Tyr Asp Ala Asp Leu Ser Ala Trp Thr Glu Tyr
        515                 520                 525

Ile Pro Tyr His Phe Arg Pro Asn Tyr His Gly Val Gly Thr Cys Ser
    530                 535                 540

Met Met Pro Lys Glu Met Gly Gly Val Val Asp Asn Ala Ala Arg Val
545                 550                 555                 560

Tyr Gly Val Gln Gly Leu Arg Val Ile Asp Gly Ser Ile Pro Pro Thr
                565                 570                 575

Gln Met Ser Ser His Val Met Thr Val Phe Tyr Ala Met Ala Leu Lys
            580                 585                 590

Ile Ser Asp Ala Ile Leu Glu Asp Tyr Ala Ser Met Gln
        595                 600                 605

Isolation of a nucleic acid molecule encoding gox can be carried out using any of the many methods of DNA isolation or preparation known in the art including, but not limited to, those described in Sambrook et al., Molecular Cloning: A Laboratory Manual, Cold Springs Laboratory, Cold Springs Harbor, New York (1989), and Mach et al., "Expression of Two Major Chitinase Genes of Trichoderma atroviride (T. harzianum P1) is Triggered by Different Regulatory Signals," Applied and Environ. Microbiol 65(5):1858-1863 (1999), which are hereby incorporated by reference in their entirety.

In another aspect of the present invention, the bioactive molecule is an avirulence protein (avr), and the recombinant nucleic acid molecule is the nucleic acid molecule encoding an avirulence protein (*avr*). Suitable *avr* nucleic acid molecules may be derived from any of the microbial or fungal species that contain an *avr* agene, including, but not limited to, *Cladosporium fulvum*. For example, a suitable bioactive molecule from *C*. *fulvum* is the avr4 protein, which is encoded by the nucleic acid molecule having SEQ ID NO:3, as follows:

atgcactaca caaccctcct cctaagcact ctcctagtcg gcacagccct cgcccaaccc 60
acaaatcctc ctgcgaagac tcccaagaag gcccccaaaa ctcaaccata caacccatgc 120
aagccccaag aagtcatcga caccaagtgt atgggtccca aggattgtct ctacccgaac 180
cccgacagtt gtacaaccta catacagtgt gtaccgctcg acgaagttgg caatgcgaag 240
cctgtggtta agccatgtcc aaaaggactg cagtggaacg ataacgttgg caagaagtgg 300
tgcgactatc caaacctgag tacgtgtccg gtaaagacgc cgcaaccgaa gccgaagaag 360
ggaggtgtcg gagggaagaa ggcgtcggtt ggacatcctg gctat 405

Another suitable avirulence protein from *C*. *fulvum* is *avr*9, which is encoded by the nucleic acid molecule having SEQ ID NO:4, as follows:

atgaagcttt ccctccttag cgtagagctt gctctcctaa ttgctactac tctcccactt 60
tgctgggcag ctgccctccc tgtaggattg ggagtcgggc tagactactg taactcaagt 120
tgtactaggg ccttcgactg tttgggtcaa tgtggcagat gcgactttca taagctacaa 180
tgtgtccact a 191

There are a wide variety of genes that may be delivered to the plant roots by the methods of the present invention and function to decrease the expression of plant disease. These genes encode or result in production of products that act in a variety of ways to decrease plant disease. For the present invention, the genes need to be introduced into a symbiotic, root-colonizing fungus having rhizosphere competence. The gene products expressed by the transgenic fungus need to be secreted into the zone of interaction between the transgenic fungus and the plant. Categories of genes suitable for the present invention include, without limitation: 1) transgenes whose gene products potentiate the defense response of plants to bacteria and fungi by increasing the antimicrobial properties of the plants; 2) genes derived from the pathogen expressed in transgenic plants, which activate the defense response and enhance the ability of the plant to recognize the pathogen; 3) transgenes whose gene products deactivate pathogen toxins or make the plant insensitive to them, and 4) transgenes against bacterial pathogens, which includes the subcategories of a) production of antibacterial proteins of non-plant origin; b) inhibition of the bacterial pathogenicity or of the virulence factors; c) improvement of plant's natural defenses; and d) artificial induction of cell death programmed on the infection site. These categories and examples for each are listed in Tables 1-4, respectively, below. Table 5, below, is a non-limiting list of exemplary genes/proteins suitable for the present invention, with their public database accession numbers. These are provided as non-limiting examples only and others will be obvious to those skilled in the art. However, in general, any gene that, when expressed in a plant or at the plants roots, results in pest or disease reduction, can be used in the present invention.

**TABLE 1**

| **Transgenes Whose Gene Products Potentiate the Defense Response of Plants to Bacteria and Fungi by Increasing the Antimicrobial Properties of the Plants** | | | | |
|---|---|---|---|---|
| **Gene** | ***Origin*** | **Activity/effect** | **Plant** | **References*** |
| Plant chitinases and glucanases | Bean, tobacco, barley, etc. | Antifungal activity. Limited resistance to *Rhizoctonia solani* and other fungi | various | various |
| Chitinase (*chiA*) | *Serratia marcescens* | Antifungal chitinases. Resistance to *Alternaria longipes* and *R*. *Solani* | Tobacco | Jones et al., "Construction of a Tra-Deletion Mutant of pAgK84 to Safeguard the Biological Control of Crown Gall," Molecular and General Genetics 212:207-214 (1988) |
| Chitosanase | *Streptomyces* sp. | Antifungal chitosanase | Tobacco | El Quakfaoui et al., "A Streptomyces Chitosanase is Active in Transgenic Tobacco," Plant Cell Reports 15:222-226 (1995) |
| Chitinase (*chi1*) | *Rhizopus oligosporus* | Chitinase involved in the autolysis. Resistance to *Sclerotinia sclerotiorum* and *Botrytis cinerea* | Tobacco | Terakawa et al., "A Fungal Chitinase Gene from Rhizopus oligosporus Confers Antifungal Activity to Transgenic Tobacco," Plant Cell Reports 16:439-443 (1997) |
| Endochitinase (*chit42*) | *Trichoderma harzianum* | Antifungal chitinases. Resistance to *A. alternata, A. solani*, *B. cinerea, R. Solani* | Tobacco, potato, apple, vine, petunia, etc. | Lorito et al., "Genes from Mycoparasitic Fungi as a Source for Improving Plant Resistance to Fungal Pathogens," Proceedings of the National Academy of Sciences of USA, Washington DC, USA 95(14):7860-7865 (1998); Bolar et al., "Endochitinase-Transgenic McIntosh Apple Lines Have Increased Resistance to Scab," Phytopathology 87(Supplement):S11 (1997) |
| Osmotin | Potato, tobacco | Inhibition of the growth and delay in the development and appearance of the symptoms with *P*. *infestans*, but not with *P*. *parasitica* var. *nicotianae* | Potato, tobacco | Liu et al., "Osmotin Overexpression in Potato Delays Development of Disease Symptoms," Proceedings of the National Academy of Sciences USA 91:1888-1892 (1994) |
| Ribosome inactivating protein (RIP) | Barley | Resistance to *Rhizoctonia solani* in combination with chitinase. | tobacco | Jach et al., "Enhanced Quantitative Resistance Against Fungal Diseases by Combinatorial Expression of Different Barley Antifungal Proteins in Transgenic Tobacco," Plant Journal 8:97-109 (1995) |
| Glucose -oxidase | *Aspergillus niger* | Produces ROS and HR. Wide spectrum resistance vs. fungi and bacteria included *Erwinia carotovora* and *P*. *infestans*. | Potato | Wu et al., "Disease Resistance Conferred by Expression of a Gene Encoding H2O2-Generating Glucose Oxidase in Transgenic Potato Plants," Plant Cell 7:1357-68 (1995) |
| Trichodiene sintasi (*Tri5*) | *Fusarium sporotrichioides* | Production of a phytoalexin sesquiturpenoid. Resistance to microbes and insects | tobacco | Hohn et al., "Expression of a Fungal Sesquiterpene Cyclase Gene in Transgenic Tobacco," Plant Physiol 97:460-462 (1991) |
| Stilbene sintasi | vine | Production of the phytoalexin resveratrol. Resistance to *Botrytis cinerea* | tobacco | Hain et al., "Disease Resistance Results from Foreign Phytoalexin Expression in a Novel Plant," Nature 361:153-156 (1993) |
| Killer toxin | *Ustilago maydis* (mycovirus) | Toxicity vs. similar species of *Ustilago*. Resistance to *U*. *maydis* | tobacco | Park et al., "High-Level Secretion of a Virally Encoded Anti-Fungal Toxin in Transgenic Tobacco Plants," Plant Molecular Biology 30:359-366 (1996) |
| Bacterial ribonuclease (barnase) with inducible promoter and barstar | *Bacillus amyloliquefaciens* | Ribonuclease localized on the infection site. Reduction of the sporulation of *P*. *infestans* | Potato | Strittmatter et al., "Inhibition of Fungal Disease Development in Plants by Engineering Controlled Cell Death," Bio/Technology 13:1085-1089 (1995) |
| Ribonuclease (*pac1*) | *Schizosaccharomyces pombe* | Degradation of the dsRNA. Resistance to viruses | tobacco | Watanabe et al., "Resistance Against Multiple Plant Viruses in Plants Mediated by a Double Stranded-RNA Specific Ribonuclease," Federation of European Biochemical Societies Letters 372:165-168 (1995) |
| Choleric toxin, subunit A | *Vibrio cholerae* | Antibacterial. Resistance to *Pseudomonas syringae* pv. *tabaci* | tobacco | Beffa et al., "Cholera Toxin Elevates Pathogen Resistance and Induces Pathogenesis-Related Expression in Tobacco," European Molecular Biology Organization Journal 14:5753-5761 (1995) |
| B.t. toxin | *Bacillus thuringiensis* | Insecticide | Mais, Potato, cotton, tomato, rice, carrot, *Arabidopsis thaliana*, etc.. | Diehn et al., "Problems That Can Limit the Expression of Foreign Genes in Plants: Lessons to be Learned From B. T. Toxin Genes," Genetic Engineering 18:83-99 (1996) |

| | | | | |
|---|---|---|---|---|
| * All references listed in Table 1 are hereby incorporated by reference in their entirety. | | | | |

**TABLE 2**

| **Genes Derived From the Pathogen Expressed in Transgenic Plants, Which Activate the Defense Response and Enhance the Ability of the Plant to Recognize the Pathogen** | | | | |
|---|---|---|---|---|
| **Gene** | **Derived from** | **Activity/effect** | **Recipient plant** | **References:*.** |
| Avirulence factor (*avr9*) | *C. fulvum* | Elicits HR. Resistance to fungi | Tomato, tobacco | De Wit, P.J.G.M., "Pathogen Avirulence and Plant Resistance: A Key Role for Recognition," Trends in Plant Science2:452-458 (1997) |
| Avirulence factor (*avr9, inf1*) | *C. fulvum, Phytophthora infestans* | Elicits HR. Resistance to potato virus X | Tomato, tobacco | Kamoun et al., "The Fungal Gene Avr9 and the Oomycete Gene inf1 Confer Avirulence to Potato Virus X on Tobacco," Molecular Plant-Microbe Interactions 12:459-462 (1999) |
| Avirulence factor (*avrB*) | *Pseudomonas syringae* | Elicits HR and Resistance genotype specific | *Arabidopsis thaliana* | Gopalan et al., "Expression of the Pseudomonas Syringae Avirulence Protein Avrb in Plant Cells Alleviates its Dependence on the Hypersensitive Response and Pathogenicity (Hrp) Secretion System in Eliciting Genotype-Specific Hypersensitive Cell Death," Plant Cell 8:1095-1105 (1996) |
| Avirulence factor (*avrPto*) | *P. syringae* pv. *tomato* | Elicits the defense response. Resistance to bacteria and viruses | Tomato | Tobias et al., "Plants Expressing the Pto Disease Resistance Gene Confer Resistance to Recombinant PVX Containing the Avirulence Gene AvrPto," Plant Journal 17:41-50 (1999) |
| Avirulence factor (*avrRpt2*) | *P. syringae* pv. *tomato* | Elicits the defense response induced by dexamethasone. Resistance to bacteria | *A. thaliana* | McNellis et al., "Glucocorticoid-Inducible Expression of a Bacterial Avirulence Gene in Transgenic Arabidopsis Induces Hypersensitive Cell Death," Plant Journal 14:247-257 (1998) |
| Virulence factor (*Ecp2*) | *C. fulvum* | Elicits HR. Resistance to *C. fulvum* | Tomato | Laugé et al., "Successful Search for a Resistance Gene in Tomato Targeted Against a Virulence Factor of a Fungal Pathogen," Proceedings of the National Academy of Sciences USA 95:9014-9018 (1998) |
| β-cryptogein | *P. cryptogea* | Elicits the defense response. Wide spectrum resistance | Potato, tobacco | Tepfer et al., "Phytophthora Resistance Through Production of a Fungal Protein Elicitor (β-cryptogein) in Tobacco," Molecular Plant-Microbe Interactions11:64-67 (1998) |
| Protonic pump (*bO*) | *Halobacterium halobium* | Activates LAR and SAR. Resistance to viruses, *P. syringae* pv. *tabaci e P*. *infestans* | Potato, tobacco | Abad et al., "Characterization of Acquired Resistance in Lesion-Mimic Transgenic Potato Expressing Bacterio-Opsin," Molecular Plant-Microbe Interactions10:635-645 (1997); |
| | | | | Mourgues et al., "Strategies to Improve Plant Resistance to Bacterial Diseases Through Genetic Engineering," Trends Biotechnol 16(5):203-10 (1998) |
| Pectate lyase (*PL3*) | *Erwinia carotovora* | Activates the defense response by producing endogenous elicitors. Resistance to *E*. *carotovora* | Potato | Wegener et al., "Pectate Lyase in Transgenic Potatoes Confers Pre-Activation of Defence Against Erwinia carotovora," Physiol Molec Plant Pathology 49:359-376 (1996) |
| Oligogalacturonide lyase | *E. carotovora* | Degrades oligosaccharides. Interferes with the recognition and suppresses the pathogenicity in the interaction Potato-*E. carotovora* | Potato | Weber et al., "Digalacturonates Induce Defense Against Soft Rot in Potato Tubers," in Seventh International Symposium on Molecular Plaht-Microbe Interactions, Edinburgh (Abstracts), p. 112 (1994) |
| Resistance to tabtoxin | *P. syringae* pv. *tabaci* | Inactivates the tabtoxin. Resistance to *P. syringae* pv. *Tabaci* | Tobacco | Anzai et al., "Transgenic Tobacco Resistant to a Bacterial Disease by the Detoxification of a Pathogenic Toxin, Mol Gen Genet," 219:492-494 (1989) |
| Ornitine carbamoyltransferase (*argK*) | *P. syringae* pv. *phaseolicola* | Resistance to bacterial phaseolotoxin. Suppresses the formation of symptoms. | Bean, tobacco | De la Fuente-Martinez et al., "Expression of a Bacterial Phaseolotoxin-Resistant Ornithyl Transcarbamylase in Transgenic Tobacco Confers Resistance to Pseudomonas Syringae pv. Phaseolicola," Biotech 10:905-909 (1992) |

| | | | | |
|---|---|---|---|---|
| * All references listed in Table 2 are hereby incorporated by reference in their entirety. | | | | |

**TABLE 3**

| **Transgenes whose gene products deactivate pathogen toxins or make the plant insensitive to them** | | | | |
|---|---|---|---|---|
| **Gene** | **Derived from** | **Activity/effect** | **Recipient plant** | **References*** |
| Resistance to tabtoxin | *P. syringae* pv. *tabaci* | Inactivates the tabtoxin. Resistance to *P*. *syringae* pv. *tabaci* | Tobacco | Anzai et al., "Transgenic Tobacco Resistant to a Bacterial Disease by the Detoxification of a Pathogenic Toxin, Molecular and General Genetics," 219:492-494 (1989) |
| Omitine carbamoyl-transferase (*argK*) | *P. syringae* pv. *phaseolicola* | Resistance to bacterial phaseolotoxin. Suppresses the formation of symptoms. | Bean, tobacco | De la Fuente-Martinez et al., "Expression of a Bacterial Phaseolotoxin-Resistant Ornithyl Transcarbamylase in Transgenic Tobacco Confers Resistance to Pseudomonas Syringae pv. Phaseolicola," Biotechnology 10:905-909 (1992) |

| | | | | |
|---|---|---|---|---|
| * All references listed in Table 3 are hereby incorporated by reference in their entirety. | | | | |

**TABLE 4**

| **Transgenes against Bacterial Pathogens** | | | | |
|---|---|---|---|---|
| Protein | Origin | Transgenic species | Resistance to pathogen listed: | Obtained resistance |

| **Production of Antibacterial Proteins of Non-Plant Origin** | | | | |
|---|---|---|---|---|
| Shiva-1 | *Hyalophora cecropia* (giant silk moth) | tobacco | *Ralstonia solanacearum* | partial |
| Shiva-1 | " | tobacco | *Pseudomonas syringae pv*. *tabaci* | " |
| AttacinE | " | Apple tree | *Erwinia amylovora* | " |
| Lysozyme | bacteriophage T4 | potato | *Erwinia carotovora* | " |
| Lysozyme | human | tobacco | *Pseudomonas syringae pv*. *tabaci* | " |
| Lactoferrin | human | tobacco | *Ralstonia solanacearum* | " |
| Tachiplesin | crab | potato | *Erwinia carotovora* | " |

| **Inhibition of the Bacterial Pathogenicity or of the Virulence Factors** | | | | |
|---|---|---|---|---|
| Resistance to tabtoxin | *Pseudomonas syringae pv*. *tabaci* | tobacco | *Pseudomonas syringae* | total |
| Omitin carbamoyl-transferase insensible to phaseolotoxin | *Pseudomonas syringae pv*. *phaseolicola* | bean | *Pseudomonas syringae pv*. *phaseolicola* | total |

| **Improvement of Plant's Natural Defenses** | | | | |
|---|---|---|---|---|
| Pectate lyase | *Erwinia carotovora* | potato | *Erwinia carotovora* | partial |
| Protein of resistance *Xa21* | Cultivars of rice resistant | rice | *Xanthomonas oryzae* | total |
| Glucose - oxidase | *Aspergillus niger* | potato | *Erwinia carotovora* | partial |
| Tionin | barley | tobacco | *Pseudomonas syringae pv*. *tabaci* | partial |

| **Artificial Induction of Cell Death Programmed on the Infection Site** | | | | |
|---|---|---|---|---|
| Bacterial opsin | *Halobacterium halobium* | tobacco | *Pseudomonas syringae pv*. *tabaci* | total |

**Table 5**

| **Gene** | **Accession Numbers** |
|---|---|
| *Photorhabdus luminescens* putative toxin transporter (mtpB) gene and putative toxin transporter (mtpD), putative toxin transporter, (mtpE), and Mcf2 (mcf2) | gi\|38482510\|gb\|AY445665.1\|[38482510] |
| *Phytophthora sojae* putative phosphatidylinositol-4-phosphate binding protein and elicitor Avr1b-1 (Avrlbl) genes, Avrlbl-IV allele | gi\|37951491\|gb\|AY426744.1\|[37951491] |
| Pseudomonas syringae avrRpt2 gene for avirulence protein | gi\|37665378\|emb\|Z21715.2\|PSAVRRPT2[37665378] |
| *Xanthomonas oryzae* pv. *oryzae* ATP sulfurylase small subunit RaxP (raxP) and RaxQ (raxQ) genes | gi\|21105248\|gb\|AF389910.1\|[21105248] |
| *Pseudomonas syringae* pv. *pisi* avrPpiC2 gene and ORF3 | gi\|11322464\|emb\|AJ277496.1\|PSY277496[11322464] |
| *Pseudomonas syringae* pv. *pisi* avrppiG1 gene | gi\|11322462\|emb\|AJ277495.1\|PSY277495[11322462] |
| *Pseudomonas syringae* pv. *phaseolicola* strain 1449A ORF1 and ORF2 genes | gi\|7677398\|gb\|AF231453.1\|AF231453[7677398] |
| *Pseudomonas syringae* pv. *phaseolicola pathovar phaseolicola* ORF1, ORF2, and insertion sequence IS100 putative transposase genes | gi\|7677393\|gb\|AF231452.1\|AF231452[7677393] |
| *Erwinia amylovora* harpin HrpN (hrpN) gene, potential ORFB-specific chaperone, virulence/avirulence effector protein homolog, probable HrpW-specific chaperone, and harpin HrpW genes, Hrp-secreted pathogenicity/avirulence protein DspE (dspE) gene | gi\|7542322\|gb\|AF083620.1\|AF083620[7542322] |
| *Erwinia amylovora* potential ORFB-specific chaperone and virulence/avirulence effector protein homolog genes | gi\|7542319\|gb\|AF083619.1\|AF083619[7542319] |
| *Pseudomonas syringae* avirulence protein (avrE) and avirulence protein (avrF) genes | gi\|2978502\|gb\|U97505.1\|PSU97505[2978502] |
| *Pseudomonas syringae* (pv. tomato) stbCBAD genes, Insertion sequence IS1240 tnpA gene and ORF | gi\|1100230\|gb\|L48985.1\|PSESTBCBAD[1100230] |
| *Xanthomonas campestris* PthN (*pthN*) gene | gi\|2384735\|gb\|AF016221.1\|AF016221[2384735] |
| *Xanthomonas campestris* pv. *malvacearum* avrb6 gene | gi\|1330243\|gb\|L06634.1\|XANRAVR[1330243 |
| *Xanthomonas oryzae* avirulence protein AvrXa10 (avrXa10) gene | gi\|123670\|gb\|U50552.1\|XOU50552[1236701] |
| *P. syringae* avirulence protein (avrC) gene | gi\|151052\|gb\|M22219.1\|PSEAVRC[151052] |
| *Pseudomonas syringae* avrRpt2 gene | gi\|151058\|gb\|L11355.1\|PSEAVRRPT[151058] |
| *Pseudomonas syringae* pv. *phaseolicola* putative alternative sigma factor (hrpL) gene, avrPphE and hrpY genes, hrpJ gene | gi\|571513\|gb\|U16817.1\|PSU16817[571513] |
| *Arabidopsis thaliana* beta-1,3-glucanase (BG3) gene | gi\|166638\|gb\|M58464.1\|ATHBG3A[166638] |
| *Arabidopsis thaliana* beta-1,3-glucanase 2 (BG2) gene | gi\|166636\|gb\|M58462.1\|ATHBG2A[166636] |
| *Arabidopsis thaliana* beta-1,3-glucanase (BG1) gene | gi\|166635\|gb\|M58463.1\|ATHBG1A[166635] |
| *P. syringae* avirulence protein (avrB) gene | gi\|151050\|gb\|M21965.1\|PSEAVRB[151050] |
| *Cysteine protease* avirulence protein avrPphB | gi\|2498171\|sp\|Q52430\|AVP3_PSESH[2498171] |
| Disease resistance protein RPP8 (Resistance to *Peronospora parasitica* protein 8) | gi \|29839585\|sp\|Q8W4J9\|RPP8_ARATH[29839585] |
| Disease resistance protein RGA2 (RGA2-blb) (Blight resistance protein RPI) | gi\|46576968\|sp\|Q7XBQ9\|RGA2_SOLBU[46576968] |
| Putative disease resistance protein RGA3 (RGA1-b1b) (Blight resistance protein B149 | gi\|46576966\|sp\|Q7XA40\|RGA3_SOLBU[46576966] |
| Putative disease resistance protein RGA4 (RGA4-blb) | 46576965\|sp\|Q7XA39\|RGA4_SOLBU[46576965] |
| Disease resistance protein RPS5 (Resistance to *Pseudomonas syringae* protein 5) (pNd3/pNd10) | gi\|46396675\|sp\|O64973\|RPS5_ARATH[46396675] |
| Disease resistance protein RPS2 (Resistance to *Pseudomonas syringae* protein 2) | gi\|30173240\|sp\|Q42484\|RPS2_ARATH[30173240] |
| COG4455: Protein of avirulence locus involved in temperature-dependent protein secretion *Microbulbifer degradans* 2-40 | gi\|48862700\|ref\|ZP_00316595.1\|[48862700] |
| Sec14-like *Melampsora lini* | gi\|45549582\|gb\|AAS67697.1\|[45549582] |
| Sec14-like *Melampsora lini* | gi\|45549580\|gb\|AAS67696.1\|[45549580] |
| Sec14-like *Melampsora lini* | gi\|45549579\|gb\|AAS67695.1\|[45549579] |
| orf-3 *Melampsora lini* vrL567-C | gi\|45504836\|gb\|AAS66954.1\|[45504836] |
| *Melampsora lini* | gi\|45504835\|gb\|AAS66953.1\|[45504835 |
| Orf-3 *Melampsora lini* | gi\|45504833\|gb\|AAS66952.1\|[45504833 |
| Truncated orf-3 *Melampsora lini* | gi\|45504832\|gb\|AAS66951.1\|[45504832] |
| Polyprotein *Melampsora lini* | gi\|45504831\|gb\|AAS66950.1\|[45504831] |
| AvrL567-B *Melampsora lini* | gi\|45504830\|gb\|AAS66949.1\|[45504830] |
| AvrL567-A *Melampsora lini* | gi\|45504829\|gb\|AAS66948.1\|[45504829] |
| ORFB *Erwinia pyrifoliae* | gi\|42766737\|gb\|AAS45455.1\|[42766737] |
| Avirulence C protein | gi\|114730\|sp\|P13836\|AVRC_PSESG[114730] |
| Avirulence B protein | gi\|114728\|sp\|P13835\|AVRBPSESG[114728] |
| RPM1-interacting protein 4 | gi\|29839550\|sp\|Q8GYN5\|RIN4_ARATH[29839550] |
| Avirulence protein - *Pseudomonas syringae* | gi\|37665379\|emb\|CAA79815.2\|[37665379] |
| Potential cysteine protease avirulence protein avrPpiC2 | gi\|34395527\|sp\|Q9F3T4\|AVP2_PSESJ[34395527] |
| Avirulence protein avrRpt2 - *Pseudomonas syringae* (strain DC3000, pv. tomato) | gi\|538696\|pir∥A40613[538696] |
| Hypothetical protein (avrc 3' region) - *Pseudomonas syringae* pv. *glycinea* | gi\|280004\|pir∥C43649[280004] |
| Avirulence protein C - *Pseudomonas syringae* pv. *glycinea* | gi\|280003\|pir∥B43649[280003] |
| Avirulence protein B - *Pseudomonas syringae* pv. *glycinea* | gi\|280002\|pir∥A43649[280002 |
| RaxQ *Xanthomonas oryzae* pv. *oryzae* | gi\|21105250\|gb\|AAM34571.1\|AF389910_2[21105250 ] |
| ATP sulfurylase small subunit RaxP *Xanthomonas oryzae* pv. *oryzae* | gi\|21105249\|gb\|AAM34570.1\|AF389910_1[21105249 ] |
| AvrPpiC2 protein *Pseudomonas syringae* pv. *pisi* | gi\|11322465\|emb\|CAC16701.1\|[11322465] |
| AvrPpiG1 protein *Pseudomonas syringae* pv. *pisi* | gi\|11322463\|emb\|CAC16700.1\|[11322463] |
| VirPphA *Pseudomonas syringae* pv. *phaseolicola* | gi\|5702216\|gb\|AAD47203.1\|AF141883_1[5702216] |
| PthN *Xanthomonas campestris* | gi\|2384736\|gb\|AAB69865.1\|[2384736] |
| Avirulence protein AvrXa10 | gi\|1236702\|gb\|AAA92974.1\|[1236702] |
| Unknown protein | gi\|1196740\|gb\|AAA88429.1\|[1196740] |
| Avirulence protein | gi\|151053\|gb\|AAA88428.1\|[151053] |
| Avirulence protein avrB | gi\|151051\|gb\|AAA25726.1\|[151051] |

In all aspects of the present invention, the desired nucleic acid molecule encoding a recombinant nucleic acid molecule capable of controlling plant disease is introduced into *Trichoderma* spp. using conventional recombinant technology. Generally, this involves inserting the nucleic acid molecule into an expression system to which the molecule is heterologous (i.e., not normally present). The introduction of a particular foreign or native gene into a host is facilitated by first introducing the gene sequence into a suitable nucleic acid vector. "Vector" is used herein to mean any genetic element, such as a plasmid, phage, transposon, cosmid, chromosome, virus, virion, etc., which is capable of replication when associated with the proper control elements and which is capable of transferring gene sequences between cells. Thus, the term includes cloning and expression vectors, as well as viral vectors.

U.S. Patent No. 4,237,224 to Cohen and Boyer, which is hereby incorporated by reference in its entirety, describes the production of expression systems in the form of recombinant plasmids using restriction enzyme cleavage and ligation with DNA ligase. These recombinant plasmids are then introduced by means of transformation and replicated in unicellular cultures including prokaryotic organisms and eukaryotic cells grown in tissue culture.

Recombinant genes may also be introduced into viruses, including vaccinia virus, adenovirus, and retroviruses, including lentivirus. Recombinant viruses can be generated by transfection of plasmids into cells infected with virus.

Suitable vectors include, but are not limited to, the following viral vectors such as lambda vector system gt11, gt WES.tB, Charon 4, and plasmid vectors such as pBR322, pBR325, pACYC177, pACYC184, pUC8, pUC9, pUC18, pUC19, pLG339, pR290, pKC37, pKC101, SV 40, pBluescript II SK +/or KS +/- (see "Stratagene Cloning Systems" Catalog (1993) from Stratagene, La Jolla, CA, which is hereby incorporated by reference in its entirety), pQE, pIH821, pGEX, pET series (F.W. Studier et. al., "Use of T7 RNA Polymerase to Direct Expression of Cloned Genes," Gene Expression Technology Vol. 185 (1990), which is hereby incorporated by reference in its entirety), and any derivatives thereof. Recombinant molecules can be introduced into cells via transformation, particularly transduction, conjugation, mobilization, or electroporation. The nucleic acid sequences are cloned into the vector using standard cloning procedures in the art, as described by Sambrook et al., Molecular Cloning: A Laboratory Manual, Cold Springs Laboratory, Cold Springs Harbor, New York (1989), which is hereby incorporated by reference in its entirety.

A variety of host-vector systems may be utilized to express the protein-encoding sequence of the present invention. Primarily, the vector system must be compatible with the host cell used. Host-vector systems include, but are not limited to the following: bacteria transformed with bacteriophage DNA, plasmid DNA, or cosmid DNA; microorganisms such as yeast containing yeast vectors; mammalian cell systems infected with virus (e.g., vaccinia virus, adenovirus, etc.); insect cell systems infected with virus (e.g., baculovirus); and plant cells infected by bacteria. The expression elements of these vectors vary in their strength and specificities. Depending upon the host-vector system utilized, any one of a number of suitable transcription and translation elements can be used.

Different genetic signals and processing events control many levels of gene expression (e.g., DNA transcription and messenger RNA ("mRNA") translation).

Depending on the vector system and host utilized, any number of suitable transcription and/or translation elements, including constitutive, inducible, and repressible promoters, as well as minimal 5' promoter elements may be used. Promoters vary in their "strength" (i.e., their ability to promote transcription). For the purposes of expressing a cloned gene, it is desirable to use strong promoters in order to obtain a high level of transcription and, hence, expression of the gene. Depending upon the host cell system utilized, any one of a number of suitable promoters may be used. For instance, when cloning in E. *coli*, its bacteriophages, or plasmids, promoters such as the T7 phage promoter, *lac* promoter, *trp* promoter, *rec*A promoter, ribosomal RNA promoter, the P_{R} and P_{L} promoters of coliphage lambda and others, including but not limited, to *lac*UV5, *omp*F, *bla*, *lpp*, and the like, may be used to direct high levels of transcription of adjacent DNA segments. Additionally, a hybrid *trp-lac*UV5 *(tac)* promoter or other *E*. *coli* promoters produced by recombinant DNA or other synthetic nucleic acid molecule techniques may be used to provide for transcription of the inserted gene. Particularly suitable for the present invention is the use of an inducible promoter that expresses the heterologous or homologous nucleic acid molecule harbored by the transgenic *Trichoderma* when it is advantageous for the biocontrol molecule to be presented to the plant system, including to the roots and seeds. An exemplary promoter for the construct of the present invention is the promoter region from the *T. atroviride* gene encoding *N-acetylhexosaminidase (nag1*). The *pnag* promoter is not constitutive, therefore, no, or only low, levels of expression are expected from genes driven by this promoter when it is grown on glucose or other repressive substrates. However, the genes driven by the *nag1* promoter are expected to be highly expressed in the absence of high levels of glucose and in the presence of fungal cell walls, chitin, target fungi, or any combination thereof (Mach et al., "Expression of Two Major Chitinase Genes of Trichoderma atroviride (T. harzianum P1) is Triggered by Different Regulatory Signals," Appl Environ Microbiol 65:1858-1863 (1999), which is hereby incorporated by reference in its entirety). For a review on maximizing gene expression see Roberts and Lauer, Methods in Enzymology, 68:473 (1979), which is hereby incorporated by reference in its entirety.

Expression vectors may be chosen which inhibit the action of the promoter unless specifically induced. In certain operons, the addition of specific inducers is necessary for efficient transcription of the inserted DNA. For example, the *lac* operon is induced by the addition of lactose or IPTG (isopropylthio-beta-D-galactoside). A variety of other operons, such as *trp*, *pro*, etc., are under different controls.

The nucleic acid molecule(s) of the present invention, a 5' nucleotide regulatory region of choice, a suitable 3' regulatory region, and if desired, a reporter gene, are incorporated into a vector-expression system of choice to prepare the nucleic acid construct of present invention using standard cloning procedures known in the art, such as described by Sambrook et al., Molecular Cloning: A Laboratory Manual, Third Edition, Cold Spring Harbor: Cold Spring Harbor Laboratory Press, New York (2001), which is hereby incorporated by reference in its entirety.

In one aspect of the present invention, a nucleic acid molecule encoding a protein of choice is inserted into a vector in the sense (i.e., 5'→3') direction, such that the open reading frame is properly oriented for the expression of the encoded protein under the control of a promoter of choice. Single or multiple nucleic acids may be ligated into an appropriate vector in this way, under the control of a suitable promoter, to prepare a nucleic acid construct of the present invention. In all aspects of the present invention, a highly suitable nucleic acid construct includes multiple nucleic acid molecules, each encoding a bioactive molecule, all of which are in sense orientation and linked, 5'-3' to one another, and all of which are under the control of one or more operably linked 5' and 3' regulatory regions for overexpression of the bioactive molecules under the appropriate conditions.

Once the isolated nucleic acid molecule encoding the desired biocontrol molecule of the present invention has been cloned into an expression vector, it is ready to be incorporated into a host. The selected molecules can be introduced into a chosen host via transformation, particularly transduction, conjugation, lipofection, protoplast fusion, mobilization, particle bombardment, or electroporation. The DNA sequences are cloned into the desired host using standard cloning procedures known in the art, as described by Sambrook et al., Molecular Cloning: a Laboratory Manual, Second Edition, Cold Springs Laboratory, Cold Springs Harbor, New York (1989), which is hereby incorporated by reference in its entirety.

Transient expression in protoplasts allows quantitative studies of gene expression since the population of cells is very high (on the order of 10⁶). To deliver DNA inside protoplasts, several methodologies have been proposed, but the most common are electroporation (Neumann et al., "Gene Transfer into Mouse Lyoma Cells by Electroporation in High Electric Fields," EMBO J. 1: 841-45 (1982); Wong et al., "Electric Field Mediated Gene Transfer," Biochem Biophys Res Commun 30;107(2):584-7 (1982); Potter et al., "Enhancer-Dependent Expression of Human Kappa Immunoglobulin Genes Introduced into Mouse pre-B Lymphocytes by Electroporation," Proc. Natl. Acad. Sci. USA 81: 7161-65 (1984), which are hereby incorporated by reference in their entirety) and polyethylene glycol (PEG) mediated DNA uptake, Sambrook et al., Molecular Cloning: A Laboratory Manual, Chap. 16, Second Edition, Cold Springs Laboratory, Cold Springs Harbor, New York (1989), which is hereby incorporated by reference in its entirety). During electroporation, the DNA is introduced into the cell by means of a reversible change in the permeability of the cell membrane due to exposure to an electric field. PEG transformation introduces the DNA by changing the elasticity of the membranes. Unlike electroporation, PEG transformation does not require any special equipment and transformation efficiencies can be equally high. Another appropriate method of introducing the gene construct of the present invention into a host cell is fusion of protoplasts with other entities, either minicells, cells, lysosomes, or other fusible lipid-surfaced bodies that contain the chimeric gene (Fraley et al., "Entrapment of a Bacterial Plasmid in Phospholipid Vesicles: Potential for Gene Transfer," Proc Natl Acad Sci USA 76(7):3348-52 (1979); Fraley et al., "Introduction of Liposome-Encapsulated SV40 DNA into Cells," J Biol Chem 255(21):10431-10435 (1980), which are hereby incorporated by reference in the entirety).

Stable transformants are preferable for the methods of the present invention, which can be achieved by using variations of the methods above as describe in Sambrook et al., Molecular Cloning: A Laborator y Manual, Chap. 16, Second Edition, Cold Springs Laboratory, Cold Springs Harbor, New York (1989), which is hereby incorporated by reference in its entirety.

Typically, an antibiotic or other compound useful for selective growth of the transformed cells only is added as a supplement to the media. The compound to be used will be dictated by the selectable marker element present in the plasmid with which the host cell was transformed. Suitable genes are those which confer resistance to gentamycin, G418, hygromycin, streptomycin, spectinomycin, tetracycline, chloramphenicol, and the like. Similarly, "reporter genes," which encode enzymes providing for production of an identifiable compound, or other markers which indicate relevant information regarding the outcome of gene delivery, are suitable. For example, various luminescent or phosphorescent reporter genes are also appropriate, such that the presence of the transgene may be ascertained visually. The selection marker employed will depend on the target species and/or host or packaging cell lines compatible with a chosen vector.

After the transgenic host cells are identified, they are grown to a desired density in cell culture media appropriate for the cell type, under conditions suitable for the maintenance and, if desired, expansion of the cell population prior to the application of the cells in accordance with the methods of the present invention.

The present invention also relates to a method of controlling plant disease. This involves applying a transgenic strain of *Trichoderma* spp., prepared as described herein above, to a plant or plant seed, where the transgenic strain of *Trichoderma* spp. includes a recombinant nucleic acid molecule encoding a bioactive molecule capable of controlling plant disease. The applying is carried out under conditions effective to control plant disease in the plant or a plant grown from the plant seed.

The transgenic *Trichoderma* of the present invention harboring one or more nucleic acid molecule(s) encoding one or more biocontrol molecules can be introduced to a plant, plants roots, or plant seed in a number of ways. In one aspect of the present invention, the transgenic *Trichoderma* is applied to the roots of a plant to control disease when the transgene encoding a bioactive molecule is expressed by the *Trichoderma* in the root environs. This application can be directly to the roots, or to the soil in which a plant or plant seed is growing or is to be planted. Several methods for application are known in the art, including, but not limited, to the following described below.

The transgenic *Trichoderma* of the present invention may be formulated or mixed to prepare granules, dusts or liquid suspensions. These can be incorporate directly into soils or planting mixes. The preparations are then mixed into the soil or planting mix volume for greenhouse applications or into the upper volume of field soil (Harman, G. E., "The Dogmas and Myths of Biocontrol. Changes in Perceptions Based on Research with Trichoderma harzianum T-22," Plant Dis. 84, 377-393 (2000), which is hereby incorporated by reference in its entirety). Equipment and procedures for such applications are well known and used in various agricultural industries. Typical rates are 0.1 to 50 kg of product containing 10⁷ to 10⁹ colony forming units (cfu) per cubic meter of planting mix or soil. The amount of formulated product can be adjusted proportionally to higher or lower levels of colony forming units. There are approximately 10¹¹ conidia per gram (Jin et al., "Development of Media and Automated Liquid Fermentation Methods to Produce Desiccation-Tolerant Propagules of Trichoderma harzianum," Biol Contr 7:267-274 (1996), which is hereby incorporated by reference in its entirety). A cfu level of between about 10⁶ and 10¹¹ is commercially useful.

Alternatively liquid suspensions (drenches) of the transgenic *Trichoderma* of the present invention can be prepared by mixing dry powder formulations into water or other aqueous carrier, including fertilizer solutions, or by diluting a liquid formulation containing the microbe in water or other aqueous solutions, including those containing fertilizers. Such solutions can then be used to water planting mixes either prior to planting or else when plants are actively growing.

Dry powders containing the transgenic *Trichoderma* of the present invention can be applied as a dust to roots, bulbs or seeds. Generally fine powders (usually 250 µm or smaller) are dusted onto seeds, bulbs or roots to the maximum carrying powder (i.e., until no more powder will adhere to the treated surface). Such powders typically contain 10⁶ to 10¹¹ cfu/g.

Liquid suspensions of products may be prepared as described above for preparing drenches suitable for in-furrow application. Such materials may be added to the furrow into which seeds are planted or small plants are transplanted. Equipment for such applications is widely used in the agricultural industry. Typical rates of application are 0.1 to 200 kg of product (10⁶ to 10¹¹ cfu/g) per hectare of field.

Granules, as described above, can be broadcast onto soil surfaces that contain growing plants, to soil at the time of planting, or onto soils into which seeds or plants will be planted. Typical rate ranges for broadcast application are from 0.1 to 1000 kg of product (10⁶ to 10¹¹ cfu/g) per hectare of field. Alternatively, spray solutions can be prepared as described above, and applied to give similar rates (Harman, G. E., "The Dogmas and Myths of Biocontrol. Changes in Perceptions Based on Research with Trichoderma harzianum T-22," Plant Dis. 84, 377-393 (2000); Lo et al., "Biological Control of Turfgrass Diseases With a Rhizosphere Competent Strain of Trichoderma harzianum," Plant Dis. 80, 736-741(1996); Lo et al., "Improved Biocontrol Efficacy of Trichoderma harzianum 1295-22 For Foliar Phases of Turf Diseases By Use of Spray Applications," Plant Dis. 81: 1132-1138 (1997), which are hereby incorporated by reference in their entirety).

In this aspect of the present invention, the transgenic strain of *Trichoderma* is applied directly to a seed, using any method of seed treatment known in the art. For example, seeds are commonly treated using slurry, film-coating or pelleting by processes well known in the trade (Harman et al., "Factors Affecting Trichoderma hamatum Applied to Seeds As a Biocontrol Agent," Phytopathology 71: 569-572 (1981); Taylor et al., "Concepts and Technologies of Selected Seed Treatments," Ann. Rev. Phytopathol. 28: 321-339 (1990), which is hereby incorporated by reference in its entirety). The beneficial microbial agents of the present invention can effectively be added to any such treatment, providing that the formulations do not contain materials injurious to the beneficial organism. Depending on the microbe in question, this may include chemical fungicides. Typically, powder or liquid formulations (10⁶ to 10¹¹ cfu/g) of the organism are suspended in aqueous suspensions to give a bioactive level of the microbe. The liquid typically contains adhesives and other materials to provide a good level of coverage of the seeds and may also improve its shape for planting or its cosmetic appeal.

For the purposes of the present invention, all treatments are designed to accomplish the same purpose, i.e., to provide a means of application that will result in effective colonization of the root by the beneficial microbe (Harman and Björkman, "Potential and Existing Uses of Trichoderma and Gliocladium For Plant Disease Control and Plant Growth Enhancement," In: Harman, G. E. and Kubicek, C. P. (ed.): Trichoderma and Gliocladium, Vol. 2. Taylor and Francis, London, pp. 229-265 (1998), which is hereby incorporated by reference in its entirety).

On one aspect of the present invention "controlling plant disease" involves conferring systemic (as opposed to localized) disease resistance to a plant. The method involves applying a transgenic strain of *Trichoderma* spp. to a plant or plant seed, where the transgenic strain of *Trichoderma* spp. has a recombinant nucleic acid molecule encoding a bioactive molecule capable of conferring systemic disease resistance to the plant or a plant grown from the plant seed. Application of the transgenic strain of *Trichoderma* spp. is carried out under conditions effective to confer systemic disease resistance to the plant or a plant grown from the plant seed. Suitable in this aspect of the present invention are the *Trichoderma* strains, heterologous or homologous nucleic acid molecules encoding a bioactive molecule, and the method of making the transgenic *Trichoderma* spp. of the present invention as described herein above.

The present invention also relates to a method of delivering a bioactive molecule to a plant or plant seed. The method involves providing a transgenic strain of *Trichoderma* spp., where the transgenic strain of *Trichoderma* spp. comprises a recombinant nucleic acid molecule encoding a bioactive molecule, and applying the transgenic strain of *Trichoderma* spp. to a plant or plant seed. Application is carried out under conditions effective to deliver the bioactive molecule to the plant or plant seed. Suitable in this aspect of the present invention are the *Trichoderma* strains, heterologous or homologous nucleic acid molecules encoding a bioactive molecule, and methods of making the transgenic *Trichoderma* as described herein above. Application of the transgenic *Trichoderma* to effect delivery of the bioactive molecule can be to plant roots, soil or plant seed, also as described in detail above, or as known in the art.

The methods and the delivery system of the present invention can be practiced with a wide variety of plants and their seeds. For all aspects of the present invention described herein above, or in the Examples below, suitable plants to which the transgenic *Trichoderma* spp. of the present invention can be applied include all varieties of dicots and monocots, including crop plants and ornamental plants. More particularly, useful crop plants include, without limitation: alfalfa, rice, wheat, barley, rye, cotton, sunflower, peanut, corn, potato, sweet potato, bean, pea, chicory, lettuce, endive, cabbage, brussel sprout, beet, parsnip, turnip, cauliflower, broccoli, radish, spinach, onion, garlic, eggplant, pepper, celery, carrot, squash, pumpkin, zucchini, cucumber, apple, pear, melon, citrus, strawberry, grape, raspberry, pineapple, soybean, tobacco, tomato, sorghum, and sugarcane. Examples of suitable ornamental plants are, without limitation, Arabidopsis *thaliana*, *Saintpaulia*, petunia, pelargonium, poinsettia, chrysanthemum, carnation, zinnia, and turfgrasses.

### EXAMPLES

### Example 1 - Properties of the Transgenic Strain

A transgenic form (SJ3 4) of strain P1 (ATCC 74058) of *T*. *atroviride* (formerly *T*. *harzianum*) was prepared as previously described (Mach et al., "Expression of Two Major Chitinase Genes of Trichoderma atroviride (T. harzianum P1) is Triggered by Different Regulatory Signals," Appl Environ Microbiol 65:1858-1863 (1999), which is hereby incorporated by reference in its entirety), except that the transgenic *Trichoderma* of the present invention contains 12-14 copies of a glucose oxidase A *(gox)* gene obtained (although not identical to) from *A*. *niger*, ATCC 9029, having the nucleic acid sequence of SEQ ID NO:1, shown above. This nucleotide sequence encodes a protein having the amino acid sequence of SEQ ID NO:2, shown above.

The construct used for transformation contains the promoter region from the *T. atroviride* gene encoding *N-acetylhexosaminidase (nag1)* fused to the nucleic acid molecule encoding gox. This construct is referred to hereafter as *pnag:gox*. The *pnag* promoter is not constitutive, therefore, no, or only low, levels of expression are expected from genes driven by this promoter when it is grown on glucose or other repressive substrates. However, the genes driven by the *nag1* promoter are expected to be highly expressed in the absence of high levels of glucose and in the presence of fungal cell walls, chitin, target fungi, or any combination thereof.

Culture filtrates from the parental or transgenic strains were obtained as follows: The strain was precultivated in shake flasks (250 rpm) in PDB (potato dextrose broth; Merck, Whitehouse Station, NJ) for 48 hrs at 25 °C, harvested by filtration through Miracloth (Calbiochem, La Jolla, CA), washed with sterile tap water, and transferred to SM media ((in g/l): KH₂PO₄, 2; (NH₄)₂SO₄, 1.4; CaCl_{2·}2H₂O, 0.3; MgSO₄·7 H₂O, 0.3; urea, 0.6; (mg/l): FeSO₄·7H₂O, 10; ZnSO₄·2H₂O, 2.8; CoCl₂·6H₂O, 3.2 (pH 5.4), and either 1.5% (w/v) glucose or colloidal chitin as carbon source). After 3 days culture filtrates were obtained by filtration through a 0.22 µm filter. The culture filtrate was dialyzed against 20 volumes of distilled water for 24 hrs at 4°C. Thereafter, concentration of culture filtrates was carried out by covering the dialysis bags with polyethylene glycol 8000, (Fluka Biochemika, Buchs, Switzerland) and leaving them for 10 hours at 4°C leading to a 20-fold concentrated solution. The filtrates were stored at -20°C with 20% (v/v) glycerol final concentration until use.

The transgenic strains were similar or identical to the parental strain in growth rate and sporulation ability.

It was previously demonstrated that the parental strain P1 of *T*. *atroviride* P1 has no glucose oxidase activity (Mach et al., "Expression of Two Major Chitinase Genes of Trichoderma atroviride (T. harzianum P1) is Triggered by Different Regulatory Signals," Appl Environ Microbiol 65:1858-1863 (1999), which is hereby incorporated by reference in its entirety). Strain SJ3 4 produced 4 [± 1] and 300 [± 19] mU/ml of glucose oxidase activity on media containing glucose or colloidal chitin, respectively. These results are consistent with previous observations that *nag* expression is induced by chitin, as well as by pathogen cell walls (Lorito et al., "Mycoparasitic Interaction Relieves Binding of the Cre1 Carbon Catabolite Repressor Protein to Promoter Sequences of the ech42 (Endochitinase-Encoding) Gene in Trichoderma harzianum," Proc Natl Acad Sci USA 93:14868-14872 (1996); Mach et al., "Expression of Two Major Chitinase Genes of Trichoderma atroviride (T. harzianum P1) is Triggered by Different Regulatory Signals," Appl Environ Microbiol 65:1858-1863 (1999), which are hereby incorporated by reference in their entirety).

To demonstrate that glucose oxidase expression was induced by direct contact with a potential host (plant pathogen), plate confrontation assays were conducted with *B. cinerea*. A red halo indicative of glucose oxidase activity was observed around *Trichoderma* strain SJ3 4 one to two hours after it contacted the host (Mach et al., "Expression of Two Major Chitinase Genes of Trichoderma atroviride (T. harzianum P1) is Triggered by Different Regulatory Signals," Appl Environ Microbiol 65:1858-1863 (1999), which is hereby incorporated by reference in its entirety). Neither the wild type nor strains transformed only with the hygromycin B resistance-conferring vector pHATα (Goldman et al., "Transformation of Trichoderma harzianum by High Voltage Electric Pulse," Current Gen 17:169-174 (1990), which is hereby incorporated by reference in its entirety) exhibited such a fast pH shift. A general acidification leading to the occurrence of red halos was observed with all strains during later stages of biocontrol (18 to 24 hours after contact). These findings indicate that induction of *nah* (naphthalene dioxygenase) gene expression caused by contact with the pathogenic host and glucose oxidase production are correlated in *T. atroviride* strain SJ3 4, and therefore the *nag1* promoter is driving the expression of the transgene as expected (Mach et al., "Expression of Two Major Chitinase Genes of Trichoderma atroviride (T. harzianum P1) is Triggered by Different Regulatory Signals," Appl Environ Microbiol 65:1858-1863 (1999), which is hereby incorporated by reference in its entirety).

Chitinolytic activity was not detected in filtrates from either *Trichoderma* strain growing on glucose or glycerol. However, there was clearly detectable chitinolytic enzyme activity in filtrates from both strains following transfer to a medium containing colloidal chitin as the sole carbon source. Under these conditions, SJ3 4 showed 55% and 70% of the N-acetyl-ß-glucosaminidase and endochitinase activities produced by the wild type, but an unmodified level of chitobiosidase activity. Therefore, transformation with several copies of the transgene in SJ3 4 produced no apparent changes in viability, but reduced expression of two biocontrol-related chitinase genes.

### Example 2 - In vitro Antifungal Abilities

*In vitro B*. *cinerea* spore germination inhibition was tested in ELISA plates essentially as previously described. A suspension of 3×10³ *Botrytis* spores and 50 µl PDB with 5 mM potassium phosphate buffer, pH 6.7, were placed in a well of an ELISA plate and 10 µl of the 20-fold concentrated culture supernatants of strain P1 or SJ3 4 grown on colloidal chitin were added. The addition of 100 mM H₂O₂ instead of culture filtrates was used as a control. The number of germinated spores was counted after 8 hours of incubation, averaged, and related to the germination percentage of a control treatment containing sterile water instead of culture filtrate, taken as 100 percent germination. Figure 1 shows the percentage of conidia (relative to a water control) of *B*. *cinerea* conidia that germinated in the presence of culture filtrates from P1 (black bars) or SJ3 4 (gray bars). As also shown in Figure 1, culture filtrates were obtained by growth in cultures in (a) the presence of glucose (low glucose oxidase production) or (b) in the presence of chitin as the carbon source (high glucose oxidase production in SJ3 4, no glucose oxidase production in P1). Also shown in Figure 1 is the germination percentage in P1 culture filtrates (c) augmented with 8 mM H₂O₂.

These results demonstrate that in the presence of chitin, where the *pnag:gox* constructs were expressed, the ability of the filtrates from SJ3 4 to inhibit germination of conidia of the plant pathogen *B. cinerea* were substantially greater than any other treatment.

For plate confrontation assays, which measure the direct parasitism of *Trichoderma* strains on target fungi, 5-mm disks of *T. atroviride* (either P1 wild-type or SJ3 4), and either *R. solani* or *P. ultimum* were placed on potato dextrose agar (PDA) at a distance from each other of 4 cm. The plates shown in Figure 2 indicate the results of these tests, with plates labeled "R" indicating *R*. *solani* as the host and "P" indicating *P. ultimum.*

Figure 2 clearly demonstrates that the zone of lysis is more rapid with the transgenic strain. The presence of *Trichoderma* spores on the far side of the plates with SJ3 4, which was not observed with P1 at the same time frame, indicates that the transgenic strain is more capable of using the target fungi as a nutrient source, and of growing across the plate, than the wild type strain. All of these facts indicate that SJ3 4 is a more aggressive parasite than P1. Furthermore, these abilities cover a wide range of plant pathogens including the Oomycete *P*. *ultimum* and the fungal Basidiomycete *R. solani.*

### Example 3 - In situ Seedling Protection

Tests were conducted to compare the ability of SJ3 4 and P1 to protect bean cultivars (*Phaseolus vulgaris* cv. Borlotto) against two different plant pathogens. For these tests, the bean seeds were coated with a 10% (w/v) suspension of Pelgel (Liphatech, Milwaukee, WI) in 20 mM potassium phosphate buffer containing 20 mM glucose. One ml of a 1×10⁸ conidia/ml suspension of *Trichoderma* was used for coating 10 g of seeds. As a control, the same suspension without *Trichoderma* was used. Pathogen-infested soil was prepared by inoculating 500 ml of PDB with *R. solani* mycelium from a 4-day old 8-cm PDA plate. Two g wet weight of the resulting biomass was used to inoculate 1 L of sterile soil. For *P. ultimum*, 1 liter of sterile soil was infested with four 3 d old 8 cm plates of the pathogen homogenized in a blender for 30 s. After 2 days the infested soil was diluted 1:4 with sterile soil and used for biocontrol assays as described above. The coated seeds were planted 4 cm deep into infested soil and their germination was monitored for 2 weeks.

In soil tests with low amounts of pathogen (*R*. *solani* 1 g biomass, *P. ultimum* 4 homogenized plates per liter of soil), the glucose oxidase producing strain provided approximately the same level of protection against *R. solani* and *P*. *ultimum* as the wild type strain. Both the number of germinated seeds and the plant height were comparable to the results previously published by Woo et al., "Disruption of the ech42 (Endochitinase-Encoding) Gene Affects Biocontrol Activity in Trichoderma harzianum P1," Molec Plant-Microbe Interact 12:419-429 (1999), which is hereby incorporated by reference in its entirety. Increase of the disease pressure by doubling the inoculum caused a complete rot of nearly all uncoated seeds and seeds coated with conidia of the wild type, as shown in Table 6, below. Instead, almost all beans treated with the glucose oxidase-producing strain could be germinated in soil infested with high amounts of either one of the two pathogens and produced plants of a size similar to the control without pathogen.

**Table 6**

| *In planta* biocontrol assays in soil with high pathogen concentration, 14 bean seeds were used for each of three independently repeated assays. | | | | | |
|---|---|---|---|---|---|
| | Number of germinated seeds | | dpc^{a} | Plant height in cm | |
| | P1 | SJ3 4 | | P1 | SJ3 4 |
| Control without | | | | | |
| | 13.3[±3.0] | 13.0[±3.3] | | 21.1[±3.1] | 22.4[±3.8] |
| pathogen | | | | | |
| *Rhizoctonia* | 1.3[±1.0] | 12.0[±2.3] | 0 | 15.9[±1.8] | 21.5[±3.0] |
| *Pythium* | 1.0[±0.7] | 12.0[±3.7] | 0 | 14.8[±2.7] | 20.0[±4.3] |

| | | | | | |
|---|---|---|---|---|---|
| ^{a} dpc (disease pressure control) indicates germination of seeds not protected by *Trichoderma.* | | | | | |

Figures 3A-B show the effect of gox transformation on seedlings. Figure 3A shows seeds treated with wild-type *Trichoderma* (P1) or the gox-containing *Trichoderma* SJ3 4, without any pathogen added. The plants shown in Figure 3B were grown from seeds treated with wild-type *Trichoderma* (P1) or the gox-containing *Trichoderma* SJ3 4, and planted in *R. solani* infested soil, while the controls contain neither the biocontrol agent nor the pathogen (control = untreated bean plants, and dpc (disease pressure control)). Figure 3B shows the positive effect seed treatment with the SJ3 4 biocontrol strain has on plant growth.

These results demonstrate clearly that strain SJ3 4 is more effective in protecting seeds and seedlings against *R. solani* than the wild type strain.

### Example 4 - Induced Systemic Resistance

Previous research has clearly demonstrated that *Trichoderma* strains added to roots and localized on roots can protect plants against foliar diseases. This is a consequence of significant changes induced by the biocontrol agents that results in systemic protection of the plant against pathogens that are located at sites that are temporally or spatially distant from the point of application, and spatially distant from the location of the *Trichoderma* strain (Harman et al., "Trichoderma Species -Opportunistic, Avirulent Plant Symbionts," Nature Microbiol Rev 2:43-56 (2004), which is hereby incorporated by reference in its entirety). Bean seeds were coated with a 10% (w/v) aqueous suspension of Pelgel containing 1x10⁸ spores/ml of *T*. *atroviride* strains wild type P1 or transgenic strain SJ3 4 plus an untreated control without *Trichoderma*, then left in an open Petri dish to air dry overnight in a laminar flow hood. Seven seeds were planted into 14 cm vases of sterile soil (one hr at 122°C), at a depth of 4 cm, incubated at 25°C with light, and maintained at high relative humidity. Leaves were inoculated at two points and four leaves per plant, using 10 plants per treatment and two replicates for each experiment. The experiments were repeated at two different times. The lesion size was calculated on the basis of: Area = π × (measured diameter A/2) × (measured diameter B/2), where "diameter" was the treatment mean for each experiment. Statistical analyses included an analysis of variance (ANOVA) of treatment means and unpaired t-tests were conducted among the *Trichoderma* seed coat treatments for each experiment. Figures 4A-C show the results of these experiments, in which biocontrol fungi were applied as seed treatments and *B. cinerea* was inoculated on the leaves of emerged plants. Figure 4A shows disease development at different times following inoculations with *B*. *cinerea* (1x10⁶ sp/ml) at 24 hr intervals. Figure 4B shows results after inoculations with different *B. cinerea* spore concentrations, and Figure 4C shows areas of foliar disease caused by *B. cinerea* 72 hr after inoculation when plants were grown in soil infested with autoclaved or live mycelia of *Rhizoctonia solani.*

Clearly, the transgenic strain has a much greater ability to reduce foliar disease and, therefore, induces a higher level of induced resistance than the wild type strain.

### Example 5 - Avr Proteins Expressed by Transgenic Trichoderma spp. are Secreted into Plants and Active Therein

The present invention is not limited only to the *gox* gene or to bean plants. The ability of symbiotic *Trichoderma* spp. to exchange molecules and affect plant metabolism has been conclusively demonstrated. The model system was the expression of resistance and hypersensitive reactions in tomato. *T. atroviride* strain P1 was transformed with the avirulence gene, avr4, from *C. fulvum,* under control of a strong constitutive promoter. *Avr4* causes a strong hypersensitive reaction in tomatoes carrying the Cf4 gene for resistance to the pathogen, but not in tomatoes carrying the Cf5 gene. This provided a very useful marker for introduction of the protein into the plant that was verified experimentally. When the transformed strain of P1 was applied to roots of young plants with the Cf4 gene, the plants died from the hypersensitive reaction, while in older plants a strong hypersensitive reaction was seen. No such reaction occurred when the transformed strain was applied to plants with the Cf5 gene, conclusively demonstrating that inoculation of plant roots resulted in transfer of the *Avr4* gene to tomato, as demonstrated by the hypersensitive reaction shown in Figures 5A-B. Figure 5A shows the root apparatus of Cf4 tomato treated with the wild type strain. Figure 5B shows treatment with transgenic avr4-expressing *Trichoderma.* Note the limited necrosis and more extensive secondary root formation in roots shown in Figure 5B.

The present findings indicate that Avr and other similar signalling molecules from *Trichoderma* and other symbiotic root colonizing microbes may be different in function and nature from those of pathogenic fungi. The reasons for this are as follows:
1. There are numerous Avr like proteins, and genes encoding them, in *Trichoderma* spp.
2. These different proteins probably act in concert.
3. The Avr proteins from pathogens such as *C. fulvum* interact very specifically with particular genotypes of plants. However, the interactions, resulting in localized and systemic resistance, and increased plant growth and yield, are not very specific and, in fact, occur across a wide range of plants (e.g., see Table 6, above).

Thus, the symbiotic plant microbes and their biochemical elicitors of improved plant phenotype are highly useful and novel new findings.

The present invention is not restricted to any specific type of plant, particular transgene, or strain of *Trichoderma.* A wide range of *Trichoderma* strains and species are symbiotic with plants and any of them are highly suitable for the present invention. Similarly, a wide range of plant species are hosts to *Trichoderma* strains. Many strains and plants have been described in (Harman et al., "Trichoderma Species -Opportunistic, Avirulent Plant Symbionts," Nature Microbiol Rev2:43-56 (2004), which is hereby incorporated by reference in its entirety). In fact, a preferred embodiment of the present invention is the use of highly rhizosphere competent strains such as *T. harzianum* strain T22 and *T. virens* strain 41. These strains effectively colonize entire root systems for the life of at least annual crops providing long-term effective and beneficial plant interactions. These have been summarized in Harman, G. E., "Myths and Dogmas of Biocontrol. Changes in Perceptions Derived from Research on Trichoderma harzianum T-22," Plant Dis84:377-393 (2000); (Harman et al., "Trichoderma Species -Opportunistic, Avirulent Plant Symbionts," Nature Microbiol Rev2:43-56 (2004), which are hereby incorporated by reference in their entirety.

Although preferred embodiments have been depicted and described in detail herein, it will be apparent to those skilled in the relevant art that various modifications, additions, substitutions, and the like can be made without departing from the spirit of the invention and these are therefore considered to be within the scope of the invention as defined in the claims which follow.

### CLAIMS OF PCT APPLICATION (PART OF DESCRIPTION FOR DIVISIONAL APPLICATION)

1. A transgenic *Trichoderma* spp. comprising a recombinant nucleic acid molecule encoding a bioactive molecule wherein the bioactive molecule is selected from the group consisting of a plant chitinase, a glucanase, a chitosanase, an endochitinase, an osmotin, a ribosome inactivating protein, a trichodiene sintasi, a stilbene sintasi, a killer toxin, a barnase, a ribonuclease, choleric toxin subunit A, a *Bacillus thuringiensis* toxin, an avirulence factor, a virulence factor, a β-cryptogein, a protonic pump, a pectate lyase, an oligogalacturonide lyase, a tabtoxin resistance protein, an ornitine carbamoyltransferase, a shiva-1, an attacinE, a lysozyme, a lactoferrin, a tachiplesin, resistance protein *Xa21,* a tionin, a toxin transporter protein, a disease resistance protein, and a bacterial opsin.
2. The transgenic *Trichoderma* spp. according to claim 1, wherein the transgenic strain of *Trichoderma* spp. comprises a plurality of nucleic acid molecules encoding a bioactive molecule.
3. The transgenic *Trichoderma* spp. according to claim 1, wherein the recombinant nucleic acid molecule is heterologous to *Trichoderma* spp.
4. The transgenic *Trichoderma* spp. according to claim 1, wherein the recombinant nucleic acid molecule is homologous to *Trichoderma* spp.
5. The transgenic *Trichoderma* spp. according to claim 1, wherein the nucleic acid molecule encoding a bioactive molecule is in an expression vector comprising a 5' regulatory region and a 3' regulatory region that collectively allow transcription and translation of the recombinant nucleic acid molecule encoding a bioactive molecule.
6. The transgenic *Trichoderma* spp. according to claim 5, wherein the 5' regulatory region is an inducible promoter region capable of driving expression of the nucleic acid molecule in the presence of fungal cell walls, chitin, or target fungi.
7. The transgenic *Trichoderma* spp. according to claim 6, wherein the inducible promoter region is a *nagl* promoter region from a *Trichoderma* spp.
8. The transgenic *Trichoderma* spp. according to claim 1, wherein the *Trichoderma* strain is rhizosphere competent.
9. The transgenic *Trichoderma* spp. according to claim 8, wherein the *Trichoderma* strain is selected from the group consisting of *T. harzianum, T. virens,* and *T. atroviride.*
10. The transgenic *Trichoderma* spp. according to claim 9, wherein the *Trichoderma* strain is *T. harzianum* strain T22.
11. The transgenic *Trichoderma* spp. according to claim 9, wherein the *Trichoderma* strain is *T. virens* strain 41.
12. A method of controlling plant disease comprising:
   applying a transgenic strain of *Trichoderma* spp. to a plant or plant seed under conditions effective to control plant disease in the plant or a plant grown from the plant seed, wherein the transgenic strain of *Trichoderma* spp. comprises a recombinant nucleic acid molecule encoding a bioactive molecule capable of controlling plant disease.
13. The method according to claim 12, wherein the transgenic strain of *Trichoderma* spp. comprises a plurality of nucleic acid molecules encoding a bioactive molecule capable of controlling plant disease.
14. The method according to claim 12, wherein the recombinant nucleic acid molecule is heterologous to *Trichoderma* spp.
15. The method according to claim 12, wherein the recombinant nucleic acid molecule is homologous to *Trichoderma* spp.
16. The method according to claim 12, wherein the nucleic acid molecule encoding a bioactive molecule capable of controlling plant disease is in an expression vector comprising a 5' regulatory region and a 3' regulatory region that collectively allow transcription and translation of the nucleic acid molecule encoding a bioactive molecule capable of controlling plant disease.
17. The method according to claim 16, wherein the 5' regulatory region is an inducible promoter region capable of driving expression of the nucleic acid molecule in the presence of fungal cell walls, chitin, or target fungi.
18. The method according to claim 17, wherein the inducible promoter region is a *nagl* promoter region from a *Trichoderma* spp.
19. The method according to claim 12, wherein the bioactive molecule is selected from the group consisting of a glucose oxidase, a plant chitinase, a glucanase, a chitosanase, an endochitinase, an osmotin, a ribosome inactivating protein, a trichodiene sintasi, a stilbene sintasi, a killer toxin, a barnase, a ribonuclease, choleric toxin subunit A, a *Bacillus thuringiensis* toxin, an avirulence factor, a virulence factor, a β-cryptogein, a protonic pump, a pectate lyase, an oligogalacturonide lyase, a tabtoxin resistance protein, an ornitine carbamoyltransferase, a shiv-1, an attacinE, a lysozyme, a lactoferrin, a tachiplesin, resistance protein *Xa21,* a tionin, a toxin transporter protein, a disease resistance protein, and a bacterial opsin.
20. The method according to claim 19, wherein the bioactive molecule is glucose oxidase.
21. The method according to claim 20, wherein the glucose oxidase either 1) is encoded by a nucleic acid molecule having a nucleotide sequence of SEQ ID NO: 1 or 2) has an amino acid sequence of SEQ ID NO:2.
22. The method according to claim 21, wherein the glucose oxidase is encoded by a nucleic acid molecule having SEQ ID NO:1.
23. The method according to claim 21, wherein the glucose oxidase has an amino acid sequence of SEQ ID NO:2.
24. The method according to claim 19, wherein the bioactive molecule is an avirulence factor.
25. The method according to claim 24, wherein the avirulence factor is selected from the group consisting of avr4, avr9, avrB, avrPt, avrRpt, avrlb-1, avr1b1-IV, avrRpt2, avrPpiC2, avrppiG1, avrE, avrF, avrb6, avrB, avrC, avrPphE, RPP8, RGA1, RGA2, RGA3, RGA-4, RGA4-b1b, RPS2, RPS5, avrL567-B, avrL567- avirc 3', RaxQ, RaxP, avrPpiC2, avrPpiG1, VirPpHA, PthN, and avrXa10.
26. The method according to claim 12, wherein the *Trichoderma* strain is rhizosphere competent.
27. The method according to claim 26, wherein the *Trichoderma* strain is selected from the group consisting of *T. harzianum, T. virens,* and *T. atroviride.*
28. The method according to claim 27, wherein the *Trichoderma* strain is *T. harzianum* strain T22.
29. The method according to claim 27, wherein the *Trichoderma* strain is *T. virens* strain 41.
30. The method according to claim 12, wherein said applying is carried out by broadcast application, liquid or dry in-furrow application, direct incorporation into soils or greenhouse planting mixes, dust or planter box treatments, or seed treatment.
31. The method according to claim 12, wherein the plant is a crop plant selected from the group consisting of alfalfa, rice, wheat, barley, rye, cotton, sunflower, peanut, corn, potato, sweet potato, bean, pea, chicory, lettuce, endive, cabbage, brussel sprout, beet, parsnip, turnip, cauliflower, broccoli, radish, spinach, onion, garlic, eggplant, pepper, celery, carrot, squash, pumpkin, zucchini, cucumber, apple, pear, melon, citrus, strawberry, grape, raspberry, pineapple, soybean, tobacco, tomato, sorghum, and sugarcane.
32. The method according to claim 12, wherein the plant is an ornamental plant selected from the group consisting of *Arabidopsis thaliana, Saintpaulia,* petunia, pelargonium, poinsettia, chrysanthemum, carnation, zinnia, and turfgrass.
33. The method according to claim 12, wherein said controlling disease involves conferring systemic disease resistance to a plant.
34. A method of delivering a bioactive molecule to a plant or plant seed, said method comprising:
   providing a transgenic strain of *Trichoderma* spp., wherein the transgenic strain of *Trichoderma* spp. comprises a recombinant nucleic acid molecule encoding a bioactive molecule; and
   applying the transgenic strain of *Trichoderma* spp. to a plant or plant seed under conditions to effective to deliver the bioactive molecule to the plant or plant seed.
35. The method according to claim 34, wherein the transgenic strain of *Trichoderma* spp. comprises a plurality of nucleic acid molecules encoding a bioactive molecule.
36. The method according to claim 34, wherein the recombinant nucleic acid molecule is heterologous to *Trichoderma* spp.
37. The method according to claim 34, wherein the recombinant nucleic acid molecule is homologous to *Trichoderma* spp.
38. The method according to claim 34, wherein the nucleic acid molecule encoding a bioactive molecule is in an expression vector comprising a 5' regulatory region and a 3' regulatory region that collectively allow transcription and translation of the nucleic acid molecule encoding a bioactive molecule.
39. The method according to claim 38, wherein the 5' regulatory region is an inducible promoter region capable of driving expression of the nucleic acid molecule in the presence of fungal cell walls, chitin, or target fungi.
40. The method according to claim 39, wherein the inducible promoter region is a *nagl* promoter region from a *Trichoderma* spp.
41. The method according to claim 34, wherein the bioactive molecule is selected from the group consisting of a glucose oxidase, a plant chitinase, a glucanase, a chitosanase, an endochitinase, an osmotin, a ribosome inactivating protein, a trichodiene sintasi, a stilbene sintasi, a killer toxin, a barnase, a ribonuclease, choleric toxin subunit A, a *Bacillus thuringiensis* toxin, an avirulence factor, a virulence factor, a β-cryptogein, a protonic pump, a pectate lyase, an oligogalacturonide lyase, a tabtoxin resistance protein, an ornitine carbamoyltransferase, a shiva-1, an attacinE, a lysozyme, a lactoferrin, a tachiplesin, resistance protein *Xa21,* a tionin, and a bacterial opsin.
42. The method according to 41, wherein the bioactive molecule is glucose oxidase.
43. The method according to claim 42, wherein the glucose oxidase either: 1) is encoded by a nucleic acid molecule having a nucleotide sequence of SEQ ID NO: 1 or 2) has an amino acid sequence of SEQ ID NO:2.
44. The method according to claim 43, wherein the glucose oxidase is encoded by a nucleic acid molecule having a nucleotide sequence of SEQ ID NO: 1.
45. The method according to claim 43, wherein the glucose oxidase has an amino acid sequence of SEQ ID NO:2.
46. The method according to claim 41, wherein the bioactive molecule is an avirulence factor.
47. The method according to claim 46, wherein the avirulence factor is selected from the group consisting of avr4, avr9, avrB, avrPt, avrRpt, avr1b-1, avr1b1-IV, avrRpt2, avrPpiC2, avrppiG1, avrE, avrF, avrb6, avrB, avrC, avrPphE, RPP8, RGA1, RGA2, RGA3, RGA-4, RGA4-b1b, RPS2, RPS5, avrL567-B, avrL567- avirc 3', RaxQ, RaxP, avrPpiC2, avrPpiG1, VirPpHA, PthN, and avrXa10.
48. The method according to claim 34, wherein said applying is carried out by broadcast application, liquid or dry in-furrow application, direct incorporation into soils or greenhouse planting mixes, dust or planter box treatments, or direct seed treatment.
49. The method according to claim 34, wherein the plant is a crop plant selected from the group consisting of alfalfa, rice, wheat, barley, rye, cotton, sunflower, peanut, corn, potato, sweet potato, bean, pea, chicory, lettuce, endive, cabbage, brussel sprout, beet, parsnip, turnip, cauliflower, broccoli, radish, spinach, onion, garlic, eggplant, pepper, celery, carrot, squash, pumpkin, zucchini, cucumber, apple, pear, melon, citrus, strawberry, grape, raspberry, pineapple, soybean, tobacco, tomato, sorghum, and sugarcane.
50. The method according to claim 34, wherein the plant is an ornamental plant selected from the group consisting of *Arabidopsis thaliana, Saintpaulia,* petunia, pelargonium, poinsettia, chrysanthemum, carnation, zinnia, and turfgrass.

## Claims

1. A method of controlling plant disease comprising:
applying a transgenic strain of *Trichoderma* spp. to a plant or plant seed under conditions effective to control plant disease in the plant or a plant grown from the plant seed, wherein the transgenic strain of *Trichoderma* spp. comprises a recombinant nucleic acid molecule encoding a bioactive molecule capable of controlling plant disease, wherein the bioactive molecule is glucose oxidase.

2. The method according to claim 1, wherein the transgenic strain of *Trichoderma* spp. comprises a plurality of nucleic acid molecules encoding a bioactive molecule capable of controlling plant disease.

3. The method according to claim 1, wherein the *Trichoderma* strain is rhizosphere competent.

4. The method according to claim 3, wherein the *Trichoderma* strain is selected from the group consisting of *T. harzianum, T. virens,* and *T. atroviride.*

5. The method according to claim 4, wherein the *Trichoderma* strain is *T. harzianum* strain T22.

6. The method according to claim 4, wherein the *Trichoderma* strain is *T. virens* strain 41.

7. The method according to claim 1, wherein said applying is carried out by broadcast application, liquid or dry in-furrow application, direct incorporation into soils or greenhouse planting mixes, dust or planter box treatments, or seed treatment.

8. The method according to claim 1, wherein said controlling disease involves conferring systemic disease resistance to a plant.

9. A method of delivering a bioactive molecule to a plant or plant seed, said method comprising:
providing a transgenic strain of *Trichoderma* spp., wherein the transgenic strain of *Trichoderma* spp. comprises a recombinant nucleic acid molecule encoding a bioactive molecule, wherein the bioactive molecule is glucose oxidase; and
applying the transgenic strain of *Trichoderma* spp. to a plant or plant seed under conditions to effective to deliver the bioactive molecule to the plant or plant seed.

10. The method according to claim 9, wherein the transgenic strain of *Trichoderma* spp. comprises a plurality of nucleic acid molecules encoding a bioactive molecule.

11. The method according to claim 1 or claim 9, wherein the recombinant nucleic acid molecule is heterologous to *Trichoderma* spp.

12. The method according to claim 1 or claim 9, wherein the recombinant nucleic acid molecule is homologous to *Trichoderma* spp.

13. The method according to claim 1 or claim 9, wherein the nucleic acid molecule encoding a bioactive molecule is in an expression vector comprising a 5' regulatory region and a 3' regulatory region that collectively allow transcription and translation of the nucleic acid molecule encoding a bioactive molecule.

14. The method according to claim 13, wherein the 5' regulatory region is an inducible promoter region capable of driving expression of the nucleic acid molecule in the presence of fungal cell walls, chitin, or target fungi.

15. The method according to claim 14, wherein the inducible promoter region is a *nagl* promoter region from a *Trichoderma* spp.

16. The method according to claim 1 or claim 9, wherein the glucose oxidase either: 1) is encoded by a nucleic acid molecule having a nucleotide sequence of SEQ ID NO: 1 or 2) has an amino acid sequence of SEQ ID NO:2.

17. The method according to claim 16, wherein the glucose oxidase is encoded by a nucleic acid molecule having a nucleotide sequence of SEQ ID NO: 1.

18. The method according to claim 16, wherein the glucose oxidase has an amino acid sequence of SEQ ID NO:2.

19. The method according to claim 1 or claim 9, wherein the bioactive molecule is an avirulence factor.

20. The method according to claim 19, wherein the avirulence factor is selected from the group consisting of avr4, avr9, avrB, avrPt, avrRpt, avr1b-1, avr1b1-IV, avrRpt2, avrPpiC2, avrppiG1, avrE, avrF, avrb6, avrB, avrC, avrPphE, RPP8, RGA1, RGA2, RGA3, RGA-4, RGA4-blb, RPS2, RPS5, avrL567-B, avrL567- avirc 3', RaxQ, RaxP, avrPpiC2, avrPpiG1, VirPpHA, PthN, and avrXa10

21. The method according to claim 1 or claim 9, wherein said applying is carried out by broadcast application, liquid or dry in-furrow application, direct incorporation into soils or greenhouse planting mixes, dust or planter box treatments, or seed treatment.

22. The method according to claim 1 or claim 9, wherein the plant is a crop plant selected from the group consisting of alfalfa, rice, wheat, barley, rye, cotton, sunflower, peanut, corn, potato, sweet potato, bean, pea, chicory, lettuce, endive, cabbage, brussel sprout, beet, parsnip, turnip, cauliflower, broccoli, radish, spinach, onion, garlic, eggplant, pepper, celery, carrot, squash, pumpkin, zucchini, cucumber, apple, pear, melon, citrus, strawberry, grape, raspberry, pineapple, soybean, tobacco, tomato, sorghum, and sugarcane.

23. The method according to claim 1 or claim 9, wherein the plant is an ornamental plant selected from the group consisting of *Arabidopsis thaliana, Saintpaulia,* petunia, pelargonium, poinsettia, chrysanthemum, carnation, zinnia, and turfgrass.
